# EUROPEAN PATENT APPLICATION

(11) **EP 4 556 493 A1**
(43) Date of publication of application: **21.05.2025**
(21) Application number: 23839901.8
(22) Date of filing: 10.07.2023
(51) Int. Cl.: C07K 16/28, C07K 14/55, C07K 14/54, C07K 14/715, C12N 15/62, A61P 35/00, A61K 39/00

(54) **CYTOKINE FUSION PROTEIN**

(30) Priority: 11.07.2022 KR 20220084866; 12.07.2022 KR 20220085847; 03.08.2022 KR 20220097012
(71) Applicant: Genuv Inc., Seoul 03175 (KR)
(72) Inventor: PARK, Heung Rok, Seoul 03175 (KR); BAE, Dong Goo, Seoul 03175 (KR); SHIN, Hyun Soo, Seoul 03175 (KR); CHOI, Eun Ju, Seoul 03175 (KR); PARK, Soo Bong, Seoul 03175 (KR); LEE, Kwang Hyun, Seoul 03175 (KR); KIM, June Myoung, Seoul 03175 (KR)
(74) Representative: J A Kemp LLP
(86) International application number: PCT/KR2023/009763
(87) International publication number: WO 2024/014808

(57) **Abstract**

The present invention is directed to a cytokine fusion protein comprising an immunocyte targeting domain and a cytokine function domain capable of specifically inducing the proliferation and/or activation of immunocytes. Specifically, the present invention is directed to a fusion protein that specifically delivers cytokines (e.g., IL-2 or IL-15) to exhausted immunocytes (e.g., exhausted T cells) in tumors to induce and increase the activation of the immunocytes. The fusion protein of the present invention can deliver a cytokine, which promotes the proliferation or activation of the immunocyte, selectively to the effector T cells, etc. in the tumor microenvironment, thereby inducing strong immune enhancement and anti-cancer response in tumors and significantly reducing systemic toxicity or side effects of cytokines through the unique structure of the cytokine function domain.

## Description

### [Technical Field]

The present invention is directed to a cytokine fusion protein comprising an immunocyte targeting domain and a cytokine function domain capable of specifically inducing the proliferation and/or activation of immunocytes. Specifically, the present invention is directed to a fusion protein that specifically delivers cytokines (e.g., IL-2 or IL-15) to exhausted immunocytes (e.g., exhausted T cells) in tumors to induce and increase the activation of the immunocytes. In addition, the present invention is directed to a polynucleotide encoding said fusion protein and a vector and a host cell comprising the polynucleotide. The present invention is also directed to a method of producing the fusion protein and a pharmaceutical composition, method, and use for treating diseases such as cancer or enhancing an immune response in a mammal with the fusion protein.

### [Background Art]

Cytokines are cell signaling molecules involved in the regulation of the immune system. For example, IL-2 is an essential cytokine mediating the proliferation and activation of T cells. IL-2 stimulates the proliferation and differentiation of T cells, induces the generation of cytotoxic T lymphocytes (CTL), differentiates peripheral lymphocytes to cytotoxic cells and lymphokine-activated killer cells (LAK), and stimulates the proliferation and activation of NK (natural killer) cells.

Due to the above actions of IL-2, IL-2 immunotherapy was expected to be effective in treating metastatic cancer, and through extensive research, human recombinant IL-2 (Proleukin^{®}, aldesleukin) was approved by the US FDA for metastatic renal cell carcinoma and metastatic melanoma. However, since Proleukin causes serious side effects such as capillary leak syndrome (CLS), the dose and patients to be treated are limited. Furthermore, it is inconvenient because it must be administered intravenously in repeated cycles of 3 times a day for 5 consecutive days due to its short half-life of no longer than 2 hours.

There are three types of IL-2 receptors: high, intermediate, and low-affinity receptors. The high-affinity receptor consists of three subunits, IL-2 receptor alpha (IL-2Rα; CD25), beta (IL-2Rβ; CD122), and gamma (IL-2Rγ; CD132). The intermediate-affinity receptor consists of IL-2Rβ and IL-2Rγ, and the low-affinity receptor consists only of IL-2Rα. Although the intermediate-affinity receptor consisting of the β and γ subunits binds IL-2 with approximately 100-fold lower affinity than the high-affinity receptor consisting of the α, β, and γ subunits, it is able to transmit signal upon IL-2 binding. The α-subunit confers high-affinity binding to the receptor but is not essential for IL-2 signaling.

Resting immune cells express only the intermediate-affinity receptor (IL-2Rβγ). Upon activation of the resting T cells by an antigen, IL-2Rα is rapidly expressed. Once IL-2Rα binds IL-2, it then engages IL-2Rβ and IL-2Rγ. IL-2 binding by the IL-2Rαβγ complex results in signal transduction and IL-2-mediated growth stimulation of effector T cells (T_{eff}) including cytotoxic T cells that destroy virus-infected cells and tumor cells.

IL-2 also mediates activation-induced cell death (AICD) in T cells. AICD is a process by which fully activated T cells undergo programmed cell death. By this process, immune tolerance can be established not only to normal self-antigens but also to persistent antigens such as tumor antigens.

In addition, IL-2 is also involved in the maintenance of peripheral CD4⁺CD25⁺ regulatory T cells (T_{reg}), which constitutively express IL-2Rα. Regulatory T cells suppress the function of cytotoxic T cells to attack self-antigens or tumor cells. Due to these pleiotropic effects, IL-2 is not optimal for inhibiting tumor growth.

A variety of approaches have been taken to reduce the toxicity of IL-2 and increase the tumor suppression effect. One approach is to inhibit IL-2 from binding to IL-2Rα, i.e., to make IL-2 bind more to the intermediate-affinity IL-2Rβγ on the T_{eff} cells rather than to the high-affinity IL-2Rαβγ receptor on the T_{reg} cells. This includes a combination of anti-IL-2 monoclonal antibody and IL-2 (Kamimura et al., J Immunol 177, 306-14 (2006); Boyman et al., Science 311, 1924-27 (2006)), IL-2 muteins with mutations at the IL-2Rα binding site (WO2012/107417, etc.), and IL-2 variants carrying polyethylene glycol (PEG) groups at the IL-2Rα binding site (WO2012/065086).

However, the IL-2 variants developed thus far have poor therapeutic indices for cancer therapy and do not confer marked anti-cancer effects even at high, toxic doses. This seems to be because the IL-2 variants, which were designed to selectively act on T_{eff} cells than on T_{reg} cells, exert their action mainly in the periphery but do not exert sufficient action in the tumor microenvironment (TME).

To overcome the above-mentioned problems, research was performed to enable IL-2 variants to act effectively in tumor tissues. An approach was to combine IL-2 with an antibody specific to tumor-associated antigens (TAA) to carry the IL-2 to the tumor tissue. This approach may deliver IL-2 to the tumor site via the TAA, but it cannot effectively present IL-2 to T_{eff} cells in the tumor. There is a need to target IL-2 selectively to T_{eff} cells among the various tumor-infiltrated lymphocytes (TIL).

In addition to IL-2, other cytokines (e.g., IL-15) can act as an important mediator that induces immune activation or suppression in the tumor microenvironment. These cytokines are secreted from immune cells and mostly exert local effects on various targets to change the microenvironment. The development of these cytokines as therapy also proves difficult due to their properties of local effect, short action, and quick disappearance.

### [Disclosure of Invention]

### [Technical Problem]

The inventors are to provide an effective cytokine therapeutic agent that can selectively activate the exhausted immunocytes in the tumor microenvironment, reducing systemic adverse effects caused by the exogenous cytokine and enhancing anti-cancer activities.

### [Solution to Problem]

The present invention is directed to a fusion protein comprising an immunocyte targeting domain and a cytokine function domain capable of specifically inducing the proliferation and/or activation of immunocytes. In order to selectively deliver a cytokine, which promotes the proliferation or activation of immunocytes, to T_{eff} cells in the TME without affecting other immune cells or endothelial cells in the periphery, the inventors designed a fusion protein having a cytokine function domain linked to an antibody domain that targets immunocytes wherein the two domains act "in-cis" on T_{eff} cells in the tumor.

The present invention provides a fusion protein comprising at least one immunocyte antigen-binding domain, an Fc domain consisting of two Fc chains, and a cytokine function domain. In an aspect of the invention, the cytokine function domain is a cytokine that promotes the proliferation or activation of immunocytes or a domain that binds to the above. The cytokine can be an exogenous cytokine introduced from the outside or an endogenous cytokine that exists in the body. The cytokine may be an IL-2 polypeptide or IL-15 polypeptide that binds to the β subunit of IL-2 receptor (IL-2Rβ) and the common γ chain receptor (also called IL-2Rγ) to induce activation of signaling through IL-2Rβγ.

In an aspect, the present invention provides a fusion protein comprising at least one immunocyte antigen-binding domain, an Fc domain consisting of two Fc chains, and a cytokine function domain, wherein the cytokine function domain comprises an α subunit polypeptide of IL-2 receptor (IL-2Rα) and IL-2 polypeptide; the IL-2Rα polypeptide and the IL-2 polypeptide are not covalently bonded to each other and are each individually linked to a different Fc chain of the Fc domain.

Since the IL-2 is preoccupied by IL-2Rα in the fusion protein of the above structure, the binding to regulatory T cells (T_{reg}), Foxp3-negative CD4 T cells, some innate lymphoid cells, or other endothelial cells expressing the high-affinity IL-2 receptor (abbreviated as "IL-2Rαβγ") is weakened, whereas it binds more specifically to T_{eff} cells such as cytotoxic CD8+ T cells, memory T cells, or NK-T cells expressing the intermediate-affinity IL-2 receptor consisting of IL-2Rβ and IL-2Rγ without IL-2Rα (abbreviated as IL-2Rβγ), thereby enhancing anti-cancer activity while significantly reducing systemic toxicity or side effects.

In an aspect, the present invention provides a fusion protein comprising at least one immunocyte antigen-binding domain, an Fc domain consisting of two Fc chains, and a cytokine function domain, wherein the cytokine function domain is a complex of IL-2Rα polypeptide and IL-2 polypeptide covalently bonded to each other, and the complex is connected to one of the Fc chains of the Fc domain via the IL-2Rα polypeptide or IL-2 polypeptide.

In an aspect, the present invention provides a fusion protein comprising at least one immunocyte antigen-binding domain, an Fc domain consisting of two Fc chains, and a cytokine function domain, wherein the cytokine function domain comprises an α subunit polypeptide of IL-15 receptor (IL-15Rα) and IL-15 polypeptide, and the IL-15α polypeptide and the IL-15 polypeptide are each individually linked to a different Fc chain of the Fc domain.

In an aspect, the present invention provides a fusion protein comprising at least one immunocyte antigen-binding domain, an Fc domain consisting of two Fc chains, and a cytokine function domain, wherein the cytokine function domain is a complex of IL-15Rα polypeptide and IL-15 polypeptide covalently bonded to each other, and the complex is connected to one of the Fc chains of the Fc domain via the IL-15Rα polypeptide or IL-15 polypeptide.

In an aspect, the present invention provides a fusion protein comprising at least one immunocyte antigen-binding domain, an Fc domain consisting of two Fc chains, and a cytokine function domain, wherein the cytokine function domain does not comprise an IL-2 polypeptide and IL-15 polypeptide but comprises only an IL-2Rα polypeptide or IL-15Rα polypeptide so as to bind to the endogenous IL-2 or IL-15 and deliver it to T_{eff} cells.

In an aspect of the invention, the IL-2Rα polypeptide includes a mature wild-type IL-2Rα polypeptide having an amino acid sequence of SEQ ID NO: 3 in the sequence listing attached to the present specification, or an IL-2-binding fragment, extracellular domain, or sushi domain thereof, or a variant thereof.

In an aspect of the invention, the IL-15Rα polypeptide includes a mature wild-type IL-15Rα polypeptide having an amino acid sequence of SEQ ID NO: 7 in the sequence listing attached to the present specification, or an IL-15-binding fragment, extracellular domain, or sushi domain thereof, or a variant thereof.

In an aspect of the invention, the IL-2Rα and IL-15Rα polypeptides may comprise an amino acid modification(s), preferably amino acid substitution(s), that enhances the binding affinity with each of their ligands, i.e., IL-2 polypeptide and IL-15 polypeptide, respectively. In an exemplary aspect, the fusion protein of the present invention comprises an IL-2Rα variant comprising at least one amino acid substitution selected from L2D, L2E, L2Q, M25L, M25I, N27A, N27T, N27I, N27Y, S39K, L42F, L42I, L42A, L42V, L45R, N57E, 1118R, H120A, H120D, H120K, H120Y, H120L, H120W, H120R, and K153G, based on the amino acid numbering of SEQ ID NO: 3. In a preferred aspect, the fusion protein of the present invention comprises an IL-2Rα variant comprising an amino acid substitution of L42I, based on the amino acid numbering of SEQ ID NO: 3.

In an aspect of the present invention, the IL-2 polypeptide includes a mature wild-type IL-2 polypeptide having the amino acid sequence of SEQ ID NO: 1 of the sequence listing attached to the present specification or a C125S variant thereof (SEQ ID NO: 2), or a circularly permutated variant thereof. In an aspect, the IL-15 polypeptide comprises a mature wild-type IL-15 polypeptide of SEQ ID NO: 6 of the sequence listing attached to the present specification or a circularly permutated variant thereof. In an aspect, the IL-2 polypeptide or IL-15 polypeptide may comprise at least one amino acid modification that enhances the binding affinity with the α-subunit of its binding receptor. In a preferred aspect, the fusion protein of the present invention comprises an IL-2 variant comprising an amino acid substitution of T37K, based on the amino acid numbering of SEQ ID NO: 1.

In an aspect of the present invention, the immunocyte antigen-binding domain comprises a Fab molecule of an antibody that specifically binds to at least one T cell target antigen or an antigen-binding fragment thereof. In an aspect of the present invention, the immunocyte antigen-binding domain is a Fab molecule of an antibody that specifically binds to an immune checkpoint protein (e.g., PD-1 (programmed cell death-1) protein) expressed on the T_{eff} cells in the tumor microenvironment or an antigen-binding fragment thereof. In an aspect of the invention, the immunocyte antigen-binding domain is a component, antibody or an antigen-binding fragment thereof that can bind to a surface marker of the CD8+ effector T cells that can confer binding specificity to the T_{eff} cells in the tumor microenvironment. In a preferred aspect, the immunocyte antigen-binding domain is a Fab molecule of an anti-PD-1 antibody or an antigen-binding fragment thereof. In an exemplary aspect, the immunocyte antigen-binding domain is a Fab molecule of pembrolizumab or nivolumab antibody or an antigen-binding fragment thereof, or a Fab molecule of an antibody or an antigen-binding fragment thereof that binds to the same epitope as pembrolizumab or nivolumab or an antigen-binding fragment thereof or competes with pembrolizumab or nivolumab or an antigen-binding fragment thereof. Preferably, the fusion protein of the present invention comprises an immunocyte antigen-binding domain comprising a Fab molecule of an anti-PD-1 antibody or an antigen-binding fragment thereof comprising complementarity determining region (CDR) 1, CDR2, and CDR3 of a heavy chain variable region comprising the amino acid sequence of SEQ ID NO: 38, 40, 42, or 44 of the sequence listing attached to the present specification, and CDR1, CDR2, and CDR3 of a light chain variable region comprising the amino acid sequence of SEQ ID NO: 39, 41, 43, or 45.

In an aspect of the invention, the immunocyte antigen-binding domain may be a multispecific binding domain comprising at least two different T cell target antigen-binding domains. In an aspect of the invention, the fusion protein of the present invention may comprise, as an additional antigen-binding domain, a Fab molecule of an antibody that specifically binds to at least one tumor cell target antigen or an antigen-binding fragment thereof.

In an aspect of the invention, the Fc domain is a homodimer in which the first and the second chain are the same, or a heterodimer in which the two chains are different from each other. In an aspect, the Fc domain is a human IgG class Fc domain. In an aspect of the invention, the Fc domain is an IgG1, IgG2, IgG3, or IgG4 subclass Fc domain. In an aspect of the invention, when the Fc domain is an IgG4 Fc domain, it preferably comprises an amino acid substitution of S228P (based on the EU numbering system as in Kabat). In an aspect of the invention, the Fc domain may lack CH2 domains and consist only of CH3 domains. Further, the Fc domain in the present invention may comprise at least one amino acid modification that reduces the binding to Fc receptors or the effector function. In an exemplary aspect, such a modification to the Fc domain includes, but is not limited to, an amino acid modification(s) at one or more positions selected from E233, L234, L235, G236, G237, N297, L328, P329, and P331 (based on EU numbering system in Kabat).

In an aspect of the invention, the Fc domain may comprise a modification(s) to promote the formation of a heterodimer between the two Fc chains. In an exemplary aspect, such a modification of Fc domain may include knob-into-hole modification, knob-into-hole disulfide modification, and the like. In a preferred aspect, the fusion protein of the present invention comprises an Fc domain variant further comprising, together with the knob-into-hole modification or knob-into-hole disulfide modification, an amino acid substitution of K360E in an Fc chain and an amino acid substitution of Q347R in the other Fc chain, or amino acid substitutions of K360E and Q347E in an Fc chain and an amino acid substitution of Q347R or amino acid substitutions of K360R and Q347R in the other Fc chain.

In an aspect of the invention, the immunocyte antigen-binding domain and the cytokine function domain may be connected to the Fc domain directly or via a linker. In an aspect of the invention, the immunocyte antigen-binding domain is connected to the N-terminal end of the Fc domain and the cytokine function domain is connected to the C-terminal end of the Fc domain, or vice versa. In an aspect of the invention, the linker is preferably a peptide linker, but is not limited thereto.

Another aspect of the present invention provides a polynucleotide encoding the fusion protein or each region thereof, a vector comprising said polypeptide, or a transformed cell into which said vector has been introduced. Yet another aspect of the present invention provides a host cell comprising said vector and a method of producing the fusion protein of the present invention by culturing said host cell.

Another aspect of the invention provides a pharmaceutical composition comprising said fusion protein as an active ingredient for the prevention, amelioration, or treatment of tumor, cancer, metastatic tumor, metastatic cancer, or infectious disease. Yet another aspect of the present invention provides a method of preventing, ameliorating, or treating tumor, cancer, metastatic tumor, metastatic cancer, or infectious disease by administering said fusion protein to an individual who needs the prevention, amelioration, or treatment of tumor, cancer, metastatic tumor, metastatic cancer, or infectious disease.

### [Advantageous Effects of Invention]

The present invention provides an effective anti-cancer cytokine therapeutic agent which selectively activates cytotoxic effector T cells in the tumor microenvironment thereby enhancing the anti-cancer activity while decreasing systemic toxicity.

### [Brief Description of Drawings]

FIGs. 1a to 1c are schematic illustrations of a fusion protein according to an embodiment of the present invention comprising IL-2 and IL-2Rα.
FIGs. 2a and 2b are schematic illustrations of a fusion protein according to an embodiment of the present invention comprising a complex of IL-2 and IL-2Rα or a complex of IL-15 and IL-15Rα.
FIGs. 3a to 3d are schematic illustrations of a fusion protein according to an embodiment of the present invention comprising IL-2Rα or IL-15Rα but not comprising IL-2 or IL-15.
FIG. 4 is the crystal structure analysis for a structure-based linker design showing the binding mode of a fusion protein according to an embodiment of the present invention to hPD-1 and IL-2Rβγ.
FIG. 5 is an SDS-PAGE result to identify Fusion Protein A according to an embodiment of the present invention.
FIGs 6a and 6b are SDS-PAGE results to identify Fusion Proteins B, C, and D according to an embodiment of the present invention.
FIG. 7 is a result of ELISA to test whether a fusion protein according to an embodiment of the present invention binds selectively to IL-2Rα (FIG. 7a) or IL-2Rβγ (FIG. 7b).
FIG. 8 is a series of graphs for the result of HEK-Blue assay showing the difference in IL-2 signaling response of a fusion protein according to an embodiment of the present invention depending on the presence or absence of PD-1 expression and the type of IL-2 receptors expressed.
FIG. 9 provides a schedule of an animal experiment to measure the *in vivo* anti-cancer effect of a fusion protein according to an embodiment of the present invention in the MC38 mouse tumor model.
FIG. 10 is a graph showing the change in tumor size (FIG. 10a), survival (FIG. 10b), and weight change (FIG. 10c) over time after administration of a fusion protein according to an embodiment of the present invention in the MC38 mouse tumor model in comparison with the results of a monotherapy of anti-PD-1 antibody and a monotherapy of heterodimeric Fc-IL2/IL2Rα.
FIG. 11 is a graph showing the change in tumor size (FIG. 11a), survival (FIG. 11b), and weight change (FIG. 11c) over time after administration of a fusion protein according to an embodiment of the present invention in the MC38 mouse tumor model in comparison with the results of the combination administration of an anti-PD-1 antibody and a heterodimeric Fc-IL2/IL2Rα.
FIG. 12 provides a schedule of an animal experiment to examine long-term memory immune response of a fusion protein according to an embodiment of the present invention.
FIG. 13 is a graph showing the change in tumor size over time after administration of a fusion protein according to an embodiment of the present invention in the long-term memory immune response experiment.
FIG. 14 is a photograph of an animal model showing the change in tumor size after administration of a fusion protein according to an embodiment of the present invention in the long-term memory immune response experiment.
FIG. 15 provides a schedule of an animal experiment to evaluate a dose-dependent anticancer effect of a single administration of a fusion protein according to an embodiment of the present invention in the MC38 mouse tumor model.
FIG. 16 is a graph showing the change in tumor size (FIG. 16a) and weight change (FIG. 16b) over time after a single administration of a fusion protein according to an embodiment of the present invention in the MC38 mouse tumor model.
FIG. 17 is a graph showing the change in tumor size (FIG. 17a) and tumor growth inhibition rate (TGI%) (FIG. 17b) over time after administration of a fusion protein according to an embodiment of the present invention in the B16F10 mouse melanoma model.
FIG. 18 is a graph showing the change in tumor size (FIG. 18a), survival (FIG. 18b), and weight change (FIG. 18c) over time after administration of a fusion protein according to an embodiment of the present invention in the Pan02 mouse pancreatic cancer model.
FIG. 19 is a graph comparing tumor size changes (FIG. 19a) and tumor growth inhibition rates (TGI%) (FIG. 19b) over time after single administrations of Fusion Proteins A and B according to an embodiment of the present invention in the MC38 mouse tumor model.
FIG. 20 is a graph of HEK-Blue test results showing differences in IL-2 signaling response by a fusion protein according to an embodiment of the present invention depending on the presence or absence of pre-treatment with an anti-PD-1 antibody.

### [Mode for the Invention]

"Protein" or "polypeptide," as used herein, refers to at least 2 amino acids covalently bonded to each other and is interpreted to encompass oligopeptides. "A protein" or "A polypeptide," as used herein, can be interpreted to include the whole or a part of the parent A protein or A polypeptide and analogs and mutants thereof. "Mutation," "modification," or "variant" is interpreted to include substitution, insertion, and/or deletion of an amino acid residue(s). Preferably, the mutants of the present invention may involve amino acid substitution. Amino acid substitution can be expressed in the order of the amino acid residue present in the parent wild-type protein, the position of the amino acid residue, and the substituting amino acid residue.

"Fusion protein," as used herein, can be interpreted to encompass a form of at least two proteins fused to each other or a complex including the same.

The terms "Programmed Cell Death-1," "PD-1," and "PD-1 protein" are used interchangeably and include variants, isoforms, species homologs of human PD-1, and analogs having at least one common epitope with PD-1.

The term "antibody," as referred to herein, includes whole antibodies and any "antigen-binding portion" or single chains thereof. An "antibody" refers to a protein comprising at least two heavy chains and two light chains inter-connected by disulfide bonds or an antigen-binding portion thereof. Each heavy chain is comprised of a heavy chain variable region and a heavy chain constant region. The heavy chain constant region is comprised of three domains, CH1, CH2, and CH3. Each light chain is comprised of a light chain variable region and a light chain constant region. The light chain constant region is comprised of one domain, CL. The heavy chain variable region (VH) and light chain variable region (VL) can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

"Antibody," as used herein, includes, but is not limited to, polyclonal, monoclonal, monospecific, polyspecific, non-specific, humanized, single-chain, chimeric, synthetic, recombinant, hybrid, mutated, and grafted antibodies. The term "antigen-binding portion" includes any antibody fragment and encompasses all antibody fragments that maintain an antigen-binding function, i.e., the ability to specifically bind to an antigen. Typically, such an antigen-binding portion includes an antigen-binding fragment.

The term "antigen-binding domain," as used herein, is a generic term for any domain that can bind to a target antigen and includes peptides, proteins, antibodies, aptamers, chemical residues, etc. When a fusion protein of the present invention comprises an antibody as an antigen-binding domain, it may comprise not only the entire antibody but also its antigen-binding portion, Fab molecule, or antigen-binding fragment as an antigen-binding domain. The term "Fab molecule" refers to a region having one constant region and one variable region of each of the heavy and light chain, and may include a part of a hinge region or may be partially modified. The term "Fab molecule" used herein is defined as including all possible antigen-binding regions of an antibody including Fab, Fab', F(ab')₂, Fab'-SH, etc.

"Antigen-binding portion," "antigen-recognizing region," "antigen-binding domain," "antigen-binding fragment," and "binding fragment," as used herein, refer to a portion of an antibody molecule comprising the amino acids that cause the specific binding between the antibody and antigen. For example, if an antigen is a large molecule, the antigen-binding fragment may bind to only a portion of the antigen. The portion of an antigen molecule that causes the specific interaction with the antigen-binding fragment is referred to as "epitope" or "antigenic determinant." Examples of "antigen-binding portion," "antigen-recognizing region," "antigen-binding domain," "antigen-binding fragment," and "binding fragment" that can be used in the present invention include, but are not limited to, Fab, Fab', F(ab')₂, Fab'-SH, Fd, Fv, scFv, (scFv)₂, scFv-Fc, (scFv)₂-Fc, dsFv, (dsFv)₂, dsFv-dsFv', VL, VH, diabody, ds-diabody, triabody, tetrabody, minibody ((scFV-CH₃)₂), nanobody, domain antibody, single domain antibody (sdAb), bivalent domain antibody, IgGdeltaCH2, Fynomer, FynomAbs (fynomers fused to antibodies), dual-affinity re-targeting (DART), AlbudAbs, BiTEs (bispecific T-cell engager), TandAbs (tandem diabodies), DAFs (dual acting Fab), two-in-one antibodies, SMIPs (small modular immunopharmaceuticals), anticalins, FN3 monobody, DARPins, Affibodies, Affilins, Affimers, Affitins, Alphabodies, Avimers, Im7, VLR, VNAR, Trimab, CrossMab, TRIDENT, binanobodies, di-sdFv, DVD-Igs (dual variable domain immunoglobulin), CovX-bodies (peptide modified antibodies), duobody, triomAbs, etc.

Said antigen-binding fragment may comprise antibody light chain variable region (VL) and heavy chain variable region (VH), but not necessarily both. For example, the so-called Fd antibody fragment consists only of the VH domain but still retains some antigen-binding function of the intact antibody.

### Fusion protein

The fusion protein of the present invention comprises at least one immunocyte antigen-binding domain, an Fc domain consisting of two Fc chains, and a cytokine function domain. In the present invention, the at least one immunocyte antigen-binding domain is a component of a fusion protein that binds to a surface marker on a desired target immunocyte such as a CD8+ effector T cell with anti-tumor activity to deliver the cytokine domain to the target cell. In the present invention, the immunocyte antigen-binding domain is preferably an anti-PD-1 antibody or an antigen-binding fragment thereof. The fusion protein of the present invention allows cytokines to be selectively transferred to tumor-infiltrated lymphocytes expressing the target due to the presence of the immunocyte antigen-binding domain, thereby minimizing the influence on NK cells or T cells in peripheral blood. In addition, the immunocyte antigen-binding domain may be, for example, a component, antibody, or antigen-binding fragment thereof that selectively binds to NKG2a, CD8a, FcRL6, CRTAM, LAG3, TIM3, CTLA4, TIGIT, etc.

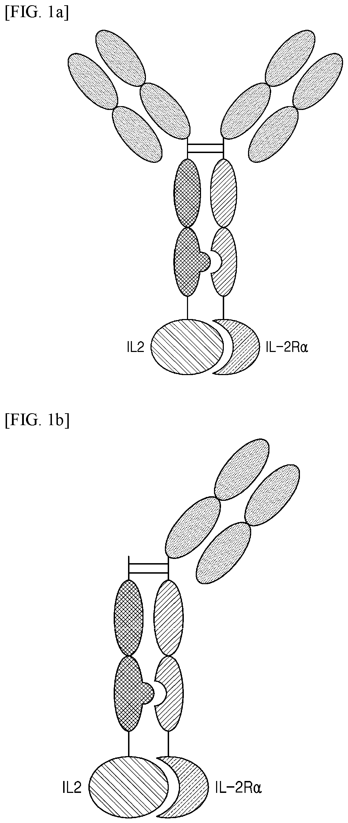

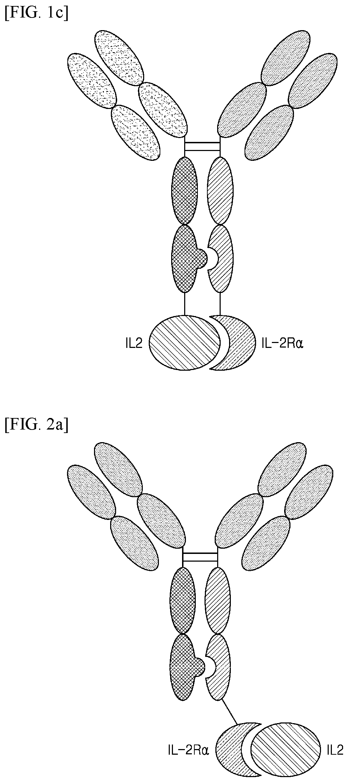

The fusion protein of the present invention may have one or two Fab arms as shown in FIGs. 1a and 1b. Alternatively, the two Fab arms may be for targets different from each other as shown in FIG. 1c. For example, if the immunocyte antigen-binding domain is an anti-PD-1 antibody or an antigen-binding fragment thereof, the fusion protein may further comprise a Fab arm for one or more targets selected from the group consisting of NKG2a, CD8a, FcRL6, CRTAM, LAG3, TIM3, CTLA4, and TIGIT. Alternatively, the fusion protein of the present invention may additionally comprise a Fab arm targeting tumor cells as an antigen-binding domain.

In one embodiment of the present invention, the cytokine function domain is designed to preferentially activate cytotoxic T_{eff} cells than T_{reg} cells. In one embodiment of the present invention, the cytokine function domain comprises a moiety, such as IL-2 polypeptide or IL-15 polypeptide, which activates signaling through IL-2Rβγ that is constitutively expressed on the exhausted T_{eff} cells. In one embodiment, the cytokine function domain also comprises IL-2Rα together with IL-2 to inhibit the IL-2 from binding to the high-affinity IL-2 receptor (IL-2Rαβγ) which is expressed on immune cells of peripheral blood or endothelial cells. IL-2 preoccupied by IL-2Rα cannot bind to a high-affinity IL-2 receptor. In an embodiment, the cytokine function domain comprises an IL-2 protein and an IL-2Rα protein, wherein the IL-2 and IL-2Rα are individually connected to the two Fc chains as shown in FIGs. 1a to 1c and are not covalently bonded to each other. IL-2 and IL-2Rα are connected to the Fc chain directly or via a linker. In one embodiment, the linker may be a peptide linker and may be, for example, (GGGX)n, (XGGG)nXGG, (GGGGX)n, (GX)n (X = A or S, n = 1, 2, 3, or 4), SGGGSGGGSGGGSGG, GGGSGGGSGGGSGG, etc., but is not limited thereto. In one embodiment, the linker may be a peptide linker consisting of approximately 5 to 20 amino acids but is not limited thereto. Linkers of any structure and length can be used as long as the fusion protein maintains the preoccupied structure of the cytokine function domain suitably and maintains the ability to deliver the cytokine function domain to the target cell. In one embodiment, the peptide linker may consist of at least one amino acid selected from the group consisting of G, S, A, P, E, T, D, and K.

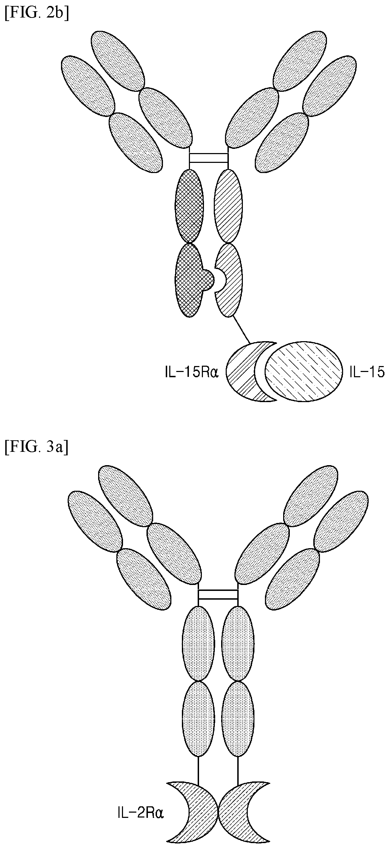

In one embodiment, the cytokine function domain may comprise IL-15 and IL-15Rα. FIG. 2b illustrates an example where the cytokine function domain comprises an IL-15 and IL-15Rα complex. In one embodiment, the cytokine function domain may comprise a covalently bonded IL-2/IL-2Rα complex (FIG. 2a) or a covalently bonded IL-15/IL-15Rα complex (FIG. 2b).

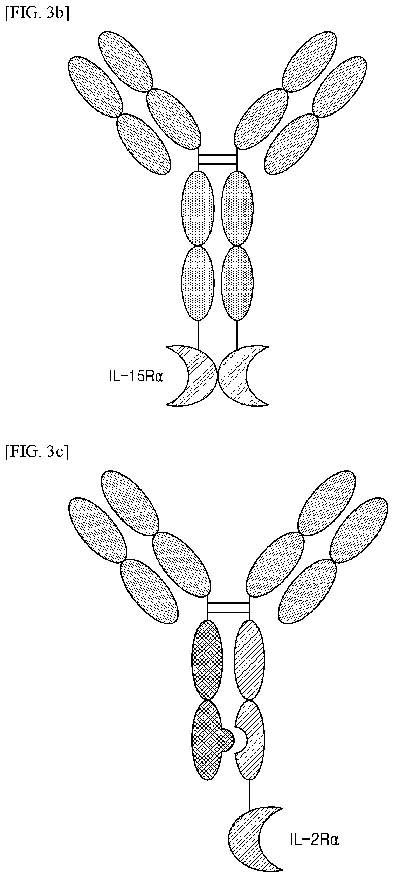

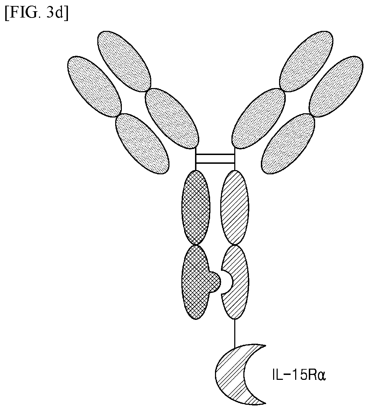

In one embodiment, the cytokine function domain does not comprise IL-2 or IL-15 but comprises IL-2Rα or IL-15Rα protein only (FIGs. 3a to 3d). In this embodiment, the IL-2Rα protein binds to endogenous IL-2 and delivers the IL-2 to cells expressing a target (e.g., T cells expressing PD-1), where it preferentially proliferates and activates T_{eff} cells on which IL-2Rβ and γ are expressed, compared to T_{reg} cells on which IL-2Rα is constitutively expressed. In this embodiment, IL-2Rα or IL-15Rα may be bound to both Fc chains to form a homodimer or may be bound to only one Fc chain to form a heterodimer.

In one embodiment, it is preferable that the cytokine function domain itself does not transmit sufficient cytokine signals, but in the form of a fusion protein combined with the immunocyte antigen-binding domain, it is selectively targeted to desired cells (cells expressing the target) to activate those cells. On the other hand, it does not activate or only weakly activates cells that express cytokine receptors but do not express targets. For example, in one embodiment, in T-cells without PD-1 expression, the cytokine function domain comprising IL-2 and IL-2Rα has a weaker binding activity to the IL-2Rβγ receptor than wild-type IL-2 and has virtually no binding activity to the IL-2Rα receptor. In contrast, the cytokine function domain in the form of a fusion protein combined with an anti-PD-1 antibody is targeted to cytotoxic CD8+ T cells that express both PD-1 and IL-2Rβγ, which then stimulates proliferation and activation of the cells through sufficient signal transmission to exhibit excellent anti-cancer activity. However, since it has little or no activity for cells without PD-1 expression, adverse effects due to cytokine activity in peripheral blood, such as capillary leakage syndrome, and systemic toxicity due to overactivated NK cells can be reduced.

### IL-2 protein

"Interleukin-2," "IL-2," "IL-2 protein," or "IL-2 polypeptide," as used herein, includes IL-2 derived from vertebrates, including mammals, such as primates (e.g., humans) and rodents (e.g., mice and rats), unless otherwise noted. This includes unprocessed IL-2 as well as any type of IL-2 processed in cells. The term also includes naturally occurring variants or "IL-2 mutants" as defined below. Full-length human IL-2 stands for mature natural length human IL-2 and is a molecule with 133 amino acids of SEQ ID NO: 1. Unprocessed human IL-2 contains an additional 20 N-terminal amino acids that are not present in the mature IL-2 molecule.

The term "IL-2 mutant" includes various types of mutant forms of IL-2 molecules including the full-length IL-2, truncated IL-2, or IL-2 linked to other molecules. The IL-2 mutant may include one or more amino acid modifications affecting the interaction with the IL-2Rα polypeptide or increasing the binding to the IL-2Rα polypeptide. This mutation may include substitution, deletion, truncation, or modification of wild-type amino acid residues. An exemplary amino acid modification that increases the binding to IL-2Rα polypeptide is T37K, based on the amino acid numbering of SEQ ID NO: 1.

In the present invention, the IL-2 protein may be a full-length human IL-2 (SEQ ID NO: 1). In addition, for example, a mutation substituting cysteine at position 125 of IL-2 of SEQ ID NO: 1 with serine (C125S, SEQ ID NO: 2) may be introduced to prevent the formation of an incorrect disulfide bond. In addition, the IL-2 protein may be a circular permutated form in which the positions of the N-terminus and C-terminus of IL-2 are changed to shorten the length of the linker.

"IL-2 polypeptide" in the present invention includes an IL-2 protein having a sequence identity of at least about 90%, at least about 91%, at least about 92%, at least about 93%, at least about 94%, at least about 95%, at least about 96%, at least about 97%, at least about 98%, or at least about 99% with the human IL-2 of SEQ ID NO: 1.

### IL-2Rα protein

"IL-2Rα protein," "IL-2Rα polypeptide," "IL-2Rα," "CD25," or "Interleukin-2 receptor α-subunit," as used herein, means an α-subunit of an IL-2 receptor derived from vertebrates, including mammals, such as primates (e.g., humans) and rodents (e.g., mice and rats), unless otherwise noted. The term includes unprocessed IL-2Rα as well as any form of IL-2Rα processed in cells. The term also includes naturally occurring variants or "IL-2Rα mutants" as defined below. Human IL-2Rα polypeptide (SEQ ID NO: 3), which is a reference for numbering in the present invention, has the amino acid sequence corresponding to positions 22 to 272 of UniProt P01589 consisting of 272 amino acids. "IL-2Rα polypeptide" in the present invention may be the IL-2Rα polypeptide having the amino acid sequence of SEQ ID NO: 3 or an IL-2-binding fragment thereof, an extracellular domain (SEQ ID NO: 4) corresponding to positions 22 to 240 of UniProt P01589 or a portion thereof, or a Sushi domain. The term "IL-2-binding fragment of an IL-2Rα polypeptide" in the present invention refers to a fragment of any length that is truncated from the C-terminus to the N-terminal direction in the extracellular domain of an IL-2Rα polypeptide and maintains the binding activity to its binding ligand, IL-2, to at least 90% or higher of the original full binding activity.

The term "IL-2Rα mutant" means an IL-2Rα protein containing a mutation(s) at one or more positions affecting the interaction with IL-2 in the various forms of IL-2Rα molecules including the extracellular domain of IL-2Rα (SEQ ID NO: 4), a truncated form of IL-2Rα, or IL-2Rα connected to another molecule. This mutation may include substitution, deletion, truncation, or modification of wild-type amino acid residues.

The "IL-2Rα mutant" may include a mutation(s) in the amino acid sequence of IL-2Rα introduced to stabilize the complex of IL-2Rα and IL-2 by increasing the affinity between them. The positions of mutations in IL-2Rα are based on the sequence of SEQ ID NO: 4. For example, the IL-2Rα mutant may comprise amino acid substitution at one or more positions selected from L2, M25, N27, S39, L42, L45, N57, I118, H120, and K153 in the extracellular domain of IL-2Rα of SEQ ID NO: 4 or a truncated form thereof. The IL-2Rα mutant may comprise at least one amino acid substitution selected from L2D, L2E, L2Q, M25L, M25I, N27A, N27T, N27I, N27Y, S39K, L42F, L42I, L42A, L42V, L45R, N57E, I118R, H120A, H120D, H120K, H120Y, H120L, H120W, H120R, and K153G. In addition, the IL-2Rα mutant may comprise at least one mutation selected from F121A, V122A, and A114D.

An exemplary IL-2Rα mutant may be a mutant of SEQ ID NO: 5 which has L42I amino acid substitution in a truncated form of an extracellular domain of IL-2Rα but is not limited thereto.

### IL-15/IL-15Rα

IL-15 is secreted by immune cells including mononuclear phagocytes after viral infection. IL-15 induces the proliferation of NK cells and other cells in the immune system and is involved in the death of virus-infected cells and cancer cells. IL-15 binds to the intermediate-affinity IL-2Rβγ receptor but binds with a much higher affinity to the IL-15Rα receptor. IL-15Rα binds to IL-2βγ to form a functional high-affinity receptor αβγ.

"Interleukin-15," "IL-15," "IL-15 protein," or "IL-15 polypeptide," as used herein, includes IL-15 derived from vertebrates, including mammals, such as primates (e.g., humans) and rodents (e.g., mice and rats), unless otherwise noted. The IL-15 polypeptide in the present invention may be the human IL-15 polypeptide having the amino acid sequence corresponding to positions 49 to 162 of Uniprot P40933 (SEQ ID NO: 6) or a variant thereof that maintains at least a portion of the activity of IL-15. The IL-15 polypeptide may include one or more amino acid modifications increasing the binding to IL-15Rα polypeptide or may be a circularly permutated form.

"IL-15Rα protein," "IL-15Rα polypeptide," "IL-15Rα," "CD215", or "interleukin-15 receptor α" means an α-subunit of IL-15 receptor derived from vertebrates, including mammals, such as primates (e.g., humans) and rodents (e.g., mice and rats), unless otherwise noted. IL-15Rα protein may be a human IL-15Rα or a truncated form or mutant thereof that maintains at least a portion of the activity of IL-15Rα. Human IL-15Rα polypeptide (SEQ ID NO: 7), which is a reference for numbering in the present invention, has the amino acid sequence corresponding to positions 31 to 267 of UniProt Q13261. IL-15Rα polypeptide in the present invention may be the IL-15Rα polypeptide having the amino acid sequence of SEQ ID NO: 7 or an IL-15-binding fragment thereof, an extracellular domain (SEQ ID NO: 8), or a Sushi domain, and may contain at least one amino acid mutation that increases the binding to its binding ligand, IL-15. The term "IL-15-binding fragment of IL-15Rα polypeptide" in the present invention refers to a fragment of any length which is truncated from the C-terminus to the N-terminal direction in the extracellular domain of IL-15Rα polypeptide and maintains the binding activity to its binding ligand, IL-15, to at least 90% or higher of the original full binding activity.

### Anti-PD-1 antibody or an antigen-binding fragment thereof

Anti-PD-1 antibody or an antigen-binding fragment thereof refers to an antibody capable of binding to PD-1, particularly a PD-1 polypeptide expressed on the cell surface or an antigen-binding fragment thereof. In some embodiments, the anti-PD-1 antibody or an antigen-binding fragment thereof binds to PD-1 with a KD of 10⁻⁷ M or less; in some embodiments, it binds to PD-1 with a KD of 10⁻⁸, 10⁻⁹, 10⁻¹⁰, or 10⁻¹¹ M or less. In some embodiments, the anti-PD-1 antibody or an antigen-binding fragment thereof binds to PD-1 even in a low pH environment with a KD of 10⁻⁹ M or less, preferably a KD of 10⁻¹⁰ M or less, more preferably a KD of 10⁻¹¹ M or less. In some embodiments, the anti-PD-1 antibody or an antigen-binding fragment thereof binds to PD-1 with a KD of 9 x 10⁻¹⁰ M or less at pH 6.0.

The anti-PD-1 antibody or an antigen-binding fragment thereof comprises a heavy chain variable region and/or a light chain variable region, wherein preferably, the heavy chain variable region comprises heavy chain complementarity-determining region 1 (HCDR1) comprising the amino acid sequence of SEQ ID NO: 9, HCDR2 comprising an amino acid sequence selected from the group consisting of SEQ ID NOs: 10 to 15, and HCDR3 comprising an amino acid sequence selected from the group consisting of SEQ ID NOs: 16 to 31, or a HCDR variant(s) with a conservative amino acid substitution(s) or no more than three amino acid mutations compared to these sequences; the light chain variable region comprises light chain complementarity-determining region 1 (LCDR1) comprising an amino acid sequence selected from the group consisting of SEQ ID NOs: 32 to 35, LCDR2 comprising the amino acid sequence of SEQ ID NO: 36, and LCDR3 comprising the amino acid sequence of SEQ ID NO: 37, or a LCDR variant(s) with a conservative amino acid substitution(s) or no more than three amino acid mutations compared to these sequences.

The anti-PD-1 antibody or an antigen-binding fragment thereof comprises a heavy chain variable region and/or a light chain variable region, wherein preferably, the heavy chain variable region comprises CDR1, CDR2, and CDR3 of a heavy chain variable region comprising an amino acid sequence as indicated by SEQ ID NO: 38, 40, 42, or 44, or the light chain variable region comprises CDR1, CDR2, and CDR3 of a light chain variable region comprising an amino acid sequence as indicated by SEQ ID NO: 39, 41, 43, or 45.

Methods of determining CDR sequences within a certain VH or VL region are known in the art, and include Kabat numbering system, Chothia numbering system, Martin numbering system, Gelfand numbering system, IMGT numbering system, and the like (*see* Dondelinger et al., Front. Immunol. (2018) 9:2278, etc.). Antibodies or functional fragments thereof according to preferred embodiments of the present invention may include CDR sequences according to the Kabat numbering system but are not limited thereto. Antibodies or functional fragments thereof comprising CDR sequences that may be defined in the variable region sequences of SEQ ID NOs: 38 to 45 according to known CDR determination methods are all included within the scope of the present invention.

The anti-PD-1 antibody or an antigen-binding fragment thereof comprises a heavy chain variable region and/or a light chain variable region, wherein preferably, the heavy chain variable region comprises a sequence as indicated by SEQ ID NO: 38, 40, 42 or 44, or a variant thereof with 1 to 10 or less amino acid mutations compared to these sequences; the light chain variable region comprises a sequence as indicated by SEQ ID NO: 39, 41, 43 or 45, or a variant with 1 to 10 or less amino acid mutations compared to these sequences.

The heavy chain variable region of the anti-PD-1 antibody or an antigen-binding fragment thereof may have an amino acid sequence having at least 75%, 80%, 85%, 86%, 87%, 88%, 89%, 90%, 91%, 92%, 93%, 94%, 95%, 96%, 97%, 98%, or 99% sequence identity to SEQ ID NO: 38, 40, 42 or 44.

The light chain variable region of the anti-PD-1 antibody or an antigen-binding fragment thereof may have an amino acid sequence having at least 75%, 80%, 85%, 86%, 87%, 88%, 89%, 90%, 91%, 92%, 93%, 94%, 95%, 96%, 97%, 98%, or 99% sequence identity to SEQ ID NO: 39, 41, 43 or 45.

A "conservative amino acid substitution" means that the amino acid residue is replaced by another amino acid residue with a side chain (R group) having similar chemical properties (e.g., charge or hydrophobicity). In general, a conservative amino acid substitution will not substantially change the functional properties of a protein. In cases where two or more amino acid sequences differ from each other by conservative substitutions, the percent similarity or the degree of similarity may be adjusted upwards to correct for the conservative nature of the substitution. Means for making this adjustment are well-known to those skilled in the art. See, e.g., Pearson (1994) Methods Mol. Biol. 24: 307-331, which is incorporated herein by reference. Examples of groups of amino acids that have side chains with similar chemical properties include 1) basic side chains: lysine, arginine, and histidine; 2) acidic side chains: aspartate and glutamate; 3) non-charged polar side chains: asparagine, glutamine, serine, threonine, tyrosine, and cysteine; 4) nonpolar side chains: glycine, alanine, valine, leucine, isoleucine, proline, phenylalanine, tryptophane, and methionine; 5) sulfur-containing side chains: cysteine and methionine; 6) aliphatic side chains: glycine, alanine, valine, leucine, isoleucine, and proline; 7) aliphatic-hydroxyl side chains: serine and threonine; 8) amide-containing side chains: asparagine and glutamine; and 9) aromatic side chains: phenylalanine, tyrosine, tryptophan, and histidine. Preferable conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256:1443-45, which is incorporated herein by reference. A "moderately conservative" replacement is any change having a nonnegative value in the PAM250 log-likelihood matrix. In the present invention, the scope of the antibody or antigen-binding fragment thereof described by referring to a specific sequence also encompasses those with a conservative substitution(s) of the sequence.

In some embodiments, the anti-PD-1 antibody or antigen-binding fragment thereof may be a recombinant antibody, preferably a murine antibody, a chimeric antibody, a humanized antibody, or a human antibody.

In some embodiments, the heavy chain constant region of the chimeric or humanized anti-PD-1 antibody may be derived from human IgG1, IgG2, IgG3, IgG4, or a mutant sequence(s) thereof, and the light chain constant region may be derived from human kappa, lambda chain, or a mutant sequence(s) thereof. For example, the heavy chain constant region may be a human IgG4 heavy chain constant region comprising the S228P mutation (SEQ ID NO. 46) and/or the light chain constant region may be a human immunoglobulin kappa constant region chain (SEQ ID NO. 47).

In some embodiments of the anti-PD-1 antibody or an antigen-binding fragment thereof, the antibody is a chimeric antibody, and the constant region is derived from a human antibody constant region or a mutant thereof.

In some embodiments of the anti-PD-1 antibody or an antigen-binding fragment thereof, the antibody is a humanized antibody, and the light chain framework region (FR) and heavy chain framework region of the antibody are derived respectively from human germline light chain and heavy chain or a mutant sequence(s) thereof.

In some embodiments of the anti-PD-1 antibody or an antigen-binding fragment thereof, the antibody is a human antibody, and the entire sequence is derived from human germline light chain and heavy chain or a mutant sequence(s) thereof.

The anti-PD-1 antibody or an antigen-binding fragment thereof may be derived from a known anti-PD-1 antibody that binds to human PD-1 (SEQ ID NO: 48). For example, it may be pembrolizumab (Keytruda^{®}), nivolumab (Opdivo^{®}), tisleizumab (BeiGene and Novartis), cemiplimab (Libtayo^{®}, Regeneron), dostarlimab (Jemperli^{®}, GSK), BCD-100, camrelizumab, genolimzumab, MED10680 (AMP-514), sasanlimab (PF-06801591), sintilimab (IBI-308), spartalizumab (PDR-001), or STI-A1110; an antibody comprising heavy chains or light chains or CDRs of these antibodies or an antigen-binding fragment thereof; or an antibody capable of binding to the same epitope as these antibodies or competing with these antibodies or an antigen-binding fragment thereof.

### Other antigen-binding domains

Immunocyte antigen-binding domains other than anti-PD-1 antibodies may be components, antibodies, or antigen-binding fragments thereof that selectively bind to, e.g., NKG2a, CD8a, FcRL6, CRTAM (CD355), LAG3, TIM3, CTLA4, TIGIT, and the like.

Examples include, but are not limited to, BMS-986315 (anti-NKG2a, WO 2020/102501), monalizumab (anti-NKG2a); mAb OKT8 or 51.1 (anti-CD8a antibody, US 10,428,155, WO 2020/060924), anti-CD8a antibodies disclosed in WO 2019/023148 or US 10,072,080; anti-FcRL6 antibody 1D8 or 7B7 disclosed in Shreeder et al. (2010) J. Immunol. 185:23, Shreeder et al. (2008) Eur. J. Immunol. 38:3159, or WO 2019/094743; anti-CRTAM antibody 5A11 disclosed in WO 2019/086878 or WO 2009/029883; relatlimab (BMS-986016, anti-LAG-3), anti-LAG-3 antibody 25F7, 26H10, 25E3, 8B7, 11F2, or 17E5 disclosed in US 2011/0150892 and WO 2014/008218, or anti-LAG-3 antibody IMP731 disclosed in US 2011/007023; ipilimumab or tremelimumab (anti-CTLA-4); TSR-022, MBG453, or LY3321367 (anti-TIM3); vibostolimab or tiragolumab (anti-TIGIT), and the like.

The fusion protein of the present invention may also comprise an antigen-binding domain targeting a tumor cell in addition to the immunocyte antigen-binding domain to form a bispecific function protein.

### Fc region

The term "Fc domain" or "Fc region," as used herein, refers to a C-terminal region containing CH2 and CH3 domains (or CH2, CH3, and CH4 domains) in the heavy chain constant region of an antibody, and is used to cover wild-type Fc regions and variants thereof. Although the boundaries of the Fc region of an IgG heavy chain can vary slightly, a human IgG heavy chain Fc region is typically defined as spanning from amino acid residue Cys226 or Pro230 to the C-terminus of a heavy chain or further comprising a hinge region in addition to the above. The numbering of amino acid residues in the Fc region is according to the EU numbering system, also called EU index of Kabat et al., Sequences of Proteins of Immunological Interest, Fifth Ed., NIH Publication, 1991, which is used when referring to a residue in a human immunoglobulin heavy chain.

The term "wild-type Fc region," as used herein, includes an amino acid sequence identical to that of a naturally occurring immunoglobulin Fc region.

The term "Fc region variant," as used herein, is a variant comprising at least one amino acid residue that is different from a wild-type Fc region and can be abbreviated as "Fc variant."

The Fc variant may have at least about 80%, preferably at least about 90% sequence identity with the parent wild-type Fc region.

The fusion protein of the present invention comprises an Fc region of an immunoglobulin consisting of two chains, a first Fc chain and a second Fc chain. For example, the Fc domain of IgG is a dimer consisting of two chains, each of which contains CH2 and CH3 IgG heavy chain constant regions.

In the present invention, the Fc region may be a homodimer or a heterodimer. In the case of a fusion protein where the first and second chains of the Fc region are connected to different protein molecules (e.g., IL-2 and IL-2Rα), it is important to inhibit pairing between the Fc chains connected to the same proteins and promote pairing between the Fc chains connected to the different proteins so as to obtain the desired fusion protein in high yields. Accordingly, it is desirable to use an Fc heterodimer modified to increase the repulsive force between the same chains while increasing the attractive force between the different chains.

In the present invention, the Fc heterodimer consists of a first Fc chain and a second Fc chain different from each other. The first Fc chain and the second Fc chain may form a tertiary structure, and they may interact with each other through a covalent bond such as a disulfide bond or a non-covalent interaction such as a hydrogen bond, an ionic bond, a van der Waals bond, or a hydrophobic interaction.

To promote the pairing of the two different chains in the Fc heterodimer of the present invention, known technology introducing mutations in the CH3 region can be used. For example, knob-into-hole (KiH) technology can be used. KiH introduces mutations into the hydrophobic interaction interface of the CH3 domains wherein in one chain, an amino acid having a large side chain is substituted with one having a small side chain, and in the other chain, a small amino acid is substituted with a large amino acid. For example, a heteromeric polypeptide described in WO 96/27011 or Ridgway, J.B. et al., Protein Eng 9 (1996) 617-621 and Merchant, A.M. et al., Nat Biotechnol 16 (1998) 677-681 may be used where T366 (based on the EU numbering system, hereinafter the same) in one chain is substituted with a large residue such as Y or W, and Y407, T394, T366, etc. in the other chain is substituted with a small residue such as T, A, or S. In one embodiment of the invention, CH3 domains of the Fc may be further modified to form a disulfide bond between the two CH3 domains by introducing cysteine (C) into the applicable positions on each CH3 domain (KiHs-s).

For example, the CH3 domain of a knob chain may comprise a T366W mutation, and the CH3 domain of a hole chain may comprise T366S, L368A, and Y407V mutations (EU numbering). In addition, by introducing E356C or S354C mutation into the CH3 domain of the knob chain and Y349C mutation into the CH3 domain of the hole chain, a disulfide bond between the CH3 domains can be formed (Merchant, A.M. et al., Nature Biotech 16 (1998) 677-681). For example, the Fc heterodimer used in the present invention may be KiHs-s.

In the present invention, one of said knob chain and hole chain further comprises an amino acid substitution of K360E and the other chain further comprises an amino acid substitution of Q347R, or one of the chains further comprises amino acid substitutions of K360E and Q347E and the other chain further comprises an amino acid substitution of Q347R or amino acid substitutions of K360R and Q347R.

A preferred Fc heterodimer to be used in this invention includes a first Fc chain comprising an amino acid substitution at T366 and a second Fc chain comprising an amino acid substitution(s) at one or more positions of T366, L368, or Y407, wherein one of the first and second Fc chains further comprises an amino acid substitution at K360, and the other chain further comprises an amino acid substitution at Q347. In addition, between the first and second Fc chains above, the chain comprising the amino acid substitution at K360 may further comprise an amino acid substitution at Q347, and/or the other chain comprising the amino acid substitution at Q347 may further comprise an amino acid substitution at K360.

Preferably, in the Fc heterodimer of the present invention,
(A1) the first Fc chain comprises amino acid substitutions of T366W and K360E, and the second Fc chain comprises amino acid substitutions of T366S, L368A, Y407V, and Q347R;
(A2) the first Fc chain comprises amino acid substitutions of T366W and Q347R, and the second Fc chain comprises amino acid substitutions of T366S, L368A, Y407V, and K360E;
(A3) the first Fc chain comprises amino acid substitutions of T366W, K360R, and Q347R, and the second Fc chain comprises amino acid substitutions of T366S, L368A, Y407V, K360E, and Q347E; or
(A4) the first Fc chain comprises amino acid substitutions of T366W, K360E, and Q347E, and the second Fc chain comprises amino acid substitutions of T366S, L368A, Y407V, K360R, and Q347R.

The Fc region in the present invention may be derived from human IgG1, IgG2, IgG3, IgG4, or their mutant sequence(s) and may further comprise other mutations to stabilize the Fc region structure or to alter the binding affinity to Fc receptors. For example, when the Fc region is derived from IgG4, a S228P mutation may be further introduced. The S228P mutation reduces Fab arm exchange in which Fab regions of two IgG4 antibodies exchange with each other. For example, an Fc region derived from IgG may comprise amino acid substitution at a position(s) selected from E233, L234, L235, G236, G237, N297, L328, P329, and P331 (EU numbering) for reduced effector function. For example, the Fc region may comprise amino acid substitution at a position(s) selected from the group consisting of L234, L235, and P329. Alternatively, the Fc region may comprise amino acid substitutions of L234A and L235A or amino acid substitutions of L234A, L235A, and P329G. Further, the Fc region in the present invention may lack CH2 domains.

Amino acid sequences of exemplary Fc chains used in the present invention are set forth in SEQ ID NOs: 49 and 50.

### Method of preparing the fusion protein

The fusion protein of the present invention may be obtained by expressing an expression vector comprising a polynucleotide sequence encoding the fusion protein in a host cell.

In the present invention, the term "polynucleotide" means an isolated nucleic acid molecule or structure consisting of a single-stranded or double-stranded deoxyribonucleotide or ribonucleotide, such as mRNA, plasmid DNA, and virus-derived RNA. The polynucleotide may be generated by combining or changing the wild-type sequences of the domains of the fusion protein and may further include elements (e.g., signal peptides) necessary for expressing the fusion protein. In addition, the polynucleotide sequence encoding the entire amino acid sequence of the fusion protein can be synthesized by PCR polymerization.

In one embodiment, when the fusion protein has a heterodimeric structure, the expression vector may comprise a first expression vector including a polynucleotide sequence encoding an amino acid sequence of a first monomer chain to which IL-2 is connected and a second expression vector including a polynucleotide sequence encoding a second monomer chain connected to IL-2Rα. In an exemplary embodiment, the first expression vector may comprise polynucleotide sequences encoding amino acid sequences of SEQ ID NOs: 51 and 57, and the second expression vector may comprise polynucleotide sequences encoding amino acid sequences of SEQ ID NOs: 51 and 58.

In one embodiment, the fusion protein may be prepared by mixing the proteins obtained after expressing the first and second expression vectors separately in different host cells or by expressing the first and second expression vectors together in a single host cell. Preferably, the fusion protein of the present invention is prepared in a single cell.

### Therapeutic administration and formulation

In one aspect, the present invention provides a pharmaceutical composition comprising a fusion protein according to the present invention and a pharmaceutically acceptable excipient or carrier. The pharmaceutical composition may be used, for example, as an immunotherapeutic agent for the treatment of tumor, cancer, metastatic tumor, or metastatic cancer. The pharmaceutical composition can also be used for the treatment of infectious diseases.

In one aspect, the pharmaceutical composition of the present invention further comprises a second therapeutic agent. In one embodiment, the second therapeutic agent is an immunomodulator, a cell growth inhibitor, a cell adhesion inhibitor, a cytotoxic agent, an apoptosis activator, or an agent that increases cell sensitivity to apoptosis inducers. In certain embodiments, the second therapeutic agent may be anticancer agents, such as microtubule disrupting agents, metabolic antagonists, topoisomerase inhibitors, DNA intercalating agents, alkylation agents, hormone therapies, kinase inhibitors, receptor antagonists, tumor cell apoptosis activators, or angiogenesis inhibitors.

Tumor, cancer, metastatic tumor, or metastatic cancer in the present invention can be, but is not limited to, non-small cell lung cancer, small cell lung cancer, renal cell cancer, kidney cancer, liver cancer, bone cancer, skin cancer, colon cancer, rectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymus cancer, leukemia, lymphoma, myeloma, mycosis fungoides, Merkel cell carcinoma, classical Hodgkin lymphoma (CHL), primary mediastinal large B-cell lymphoma, T cell/histiocyte rich B-cell lymphoma, Epstein-Barr virus (EBV)-positive and -negative posttransplant lymphoproliferative disorder (PTLD), EBV associated diffuse large B cell lymphoma (DLBCL), plasmablastic lymphoma, extranodal NK/T-cell lymphoma, nasopharyngeal carcinoma, human herpes virus 8 (HHV8)-associated primary effusion lymphoma, other blood cancers including Hodgkin lymphoma, and neoplasms in the central nervous system including primary central nervous system (CNS) lymphoma, spinal tumor, and brainstem glioma.

Infectious disease in the present invention can be, but is not limited to, chronic viral infection including viral infection of hepatitis B, hepatitis C, herpes virus, Epstein-Barr virus, HIV, cytomegalovirus, herpes simplex virus type 1, herpes simplex virus type 2, human papilloma virus, adenovirus, Kaposi West sarcoma associated with herpes virus epidemics, thin ring virus (Torquetenovirus), JC virus, or BK virus.

The pharmaceutical composition according to the present invention may be used to treat early or late-stage symptoms of cancer. In one aspect, the pharmaceutical composition can be used to treat metastatic cancer. The pharmaceutical composition is useful for reducing, inhibiting, or shrinking tumor growth in both solid tumors and blood cancer. In certain embodiments, the treatment of the pharmaceutical composition causes at least 50%, at least 60%, at least 70%, at least 80%, or at least 90% of tumor reduction in the subject. In certain embodiments, the pharmaceutical composition may be used to prevent the recurrence of a tumor. In certain embodiments, the pharmaceutical composition is useful for prolonging the overall survival of subjects with cancer. In some embodiments, the pharmaceutical composition is useful for reducing toxicity caused by chemotherapy or radiation therapy while maintaining long-term survival in patients with cancer.

In another embodiment, the pharmaceutical composition may be used as an adjuvant therapy in addition to other preparation or therapy known to those skilled in the art that is useful for the treatment of cancer.

The pharmaceutical composition in accordance with this invention can be administered with suitable carriers, excipients, and other agents incorporated into the formulation to improve transport, delivery, resistance, and the like. A number of suitable formulations can be found in formularies known to all pharmaceutical chemists *(see* Remington's Pharmaceutical Sciences, Mack Publishing Company, Easton, PA). These formulations include, for example, powder, paste, ointment, jelly, wax, oil, liquid, liquid (cations or anions) containing vesicle (e.g., LIPOFECTIN^{™}), DNA conjugates, anhydrous absorption paste, water-in-oil and oil-in-water emulsions, emulsion carbowax (polyethylene glycols of various molecular weights), semi-solid gels, and semi-solid mixtures including carbowax *(see,* Powell et al. "Compendium of excipients for parenteral formulations" PDA (1998) J Pharm Sci Technol 52:238-311).

The dose of the fusion protein may vary depending on the age and weight of the subject, target disease, conditions, and route of administration. The fusion protein of the present invention is suitably administered to the patient at once or over a series of treatments. Depending on the type and severity of the disease, the initial candidate dose of the fusion protein may be about 1 µg/kg to 15 mg/kg (e.g., 0.1 mg/kg to 10 mg/kg), whether by one or more separate administrations or continuous infusion. A typical daily dose may range from about 1 µg/kg to 100 mg/kg or more depending on the aforementioned factors. For repeated administrations over several days or longer, depending on the patient's condition, the treatment is generally sustained until a desired suppression of disease symptoms occurs. An exemplary dose may be from about 0.005 mg/kg to about 10 mg/kg. In another non-limiting example, the dose per administration may also include about 1 µg/kg, about 5 µg/kg, about 10 µg/ kg, about 50 µg/kg, about 100 µg/kg, about 200 µg/kg, about 350 µg/kg, about 500 µg/kg, about 1 mg/kg, about 5 mg/kg, about 10 mg/kg, about 50 mg/kg, about 100 mg/kg, about 200 mg/kg, about 350 mg/kg, about 500 mg/kg to about 1000 mg/kg or more, and any range that may be inferred from the above. Therefore, at least one dose of about 0.5 mg/kg, 2.0 mg/kg, 5.0 mg/kg, or 10 mg/kg (or any combination thereof) may be administered to the above patient. Such doses may be administered intermittently, e.g., every week or every three weeks (e.g., such that the patient receives from about two to about twenty, or e.g., about six doses of the fusion protein). An initial higher loading dose, followed by one or more lower doses may be administered.

The pharmaceutical composition of the present invention may be administered by various delivery systems, such as encapsulation in liposomes, microparticles, microcapsules, recombinant cells capable of expressing mutant viruses, and receptor-mediated endocytosis (*see,* e.g., Wu et al. (1987) J Biol. Chem. 262:4429-4432). The administration route includes, but is not limited to, intradermal, transdermal, intramuscular, intraperitoneal, intravenous, subcutaneous, intranasal, epidural, and oral. The composition may be administered by any conventional route, for example, by infusion or bolus injection or by absorption through epithelial or mucocutaneous lining (e.g., oral mucosa, rectal and intestinal mucosa, and the like) and may be administered with other biologically active agents. The administration may be systemic or local administration.

The pharmaceutical composition of the present invention may also be delivered in vesicles, in particular, liposomes (*see,* for example, Langer (1990) Science 249:1527-1533). The use of nanoparticles carrying a fusion protein of the present invention may also be considered. Antibody-conjugated nanoparticles can be used for both therapeutic and diagnostic purposes. The antibody-conjugated nanoparticles and preparation methods and use thereof are described in detail in Arruebo, M. et al. 2009, "Antibody-conjugated nanoparticles for biomedical applications" in J. Nanomat. Volume 2009, Article ID 439389, 24 pages, doi: 10.1155/2009/439389, which is incorporated herein by reference. Nanoparticles can be developed and conjugated to antibodies contained in a pharmaceutical composition to target tumor cells or autoimmune tissue cells or virus-infected cells. Nanoparticles for drug delivery are also described, for example, in US 8257740 or US 8246995, the full text of which is incorporated herein by reference.

In certain embodiments, the pharmaceutical composition of the present invention may be delivered in a controlled release system. In one embodiment, a pump may be used. In yet another embodiment, a polymeric material may be used. In another embodiment, the controlled release system may be placed in proximity to the therapeutic target, thus requiring only a fraction of the systemic dose. Injectable formulations may include dosage forms for intravenous, subcutaneous, intradermal, intracranial, intraperitoneal, and intramuscular injection, drip infusion, and the like. These injectable formulations may be prepared by officially known methods. The injectable formulation may be prepared, for example, by dissolving, suspending, or emulsifying the antibody or a salt thereof described above in a sterile aqueous medium or an oily medium commonly used for injection. Aqueous media that can be used for injection are, for example, physiological saline, isotonic solutions containing glucose, other adjuvants, etc., which can be used in combination with appropriate solubilizing agents, such as alcohols (e.g., ethanol), polyols (e.g., propylene glycol, polyethylene glycol), nonionic surfactants [e.g., polysorbate 80, HCO-50 (polyoxyethylene (50 mol) adduct) of hydrogenated castor oil], and so on. Oily media that can be used are, for example, sesame oil, soybean oil, etc., which can be combined with solubilizing agents such as benzyl benzoate, benzyl alcohol, and so on. The prepared injection solution is preferably filled into a suitable ampoule.

The pharmaceutical composition of the present invention can be delivered subcutaneously or intravenously using standard needles and syringes. In addition, in the case of subcutaneous transfer, it is easy to transfer the pharmaceutical composition using a pen delivery device. These pen delivery devices can be reused or can be disposable. Repeat-use pen-type delivery devices typically utilize replaceable cartridges containing a pharmaceutical composition. Once all the pharmaceutical composition in the cartridge is administered and the cartridge is emptied, it can be discarded and replaced with a new cartridge containing the composition. The pen delivery device can be used repeatedly. In the disposable pen-type transfer device, there is no replaceable cartridge. Instead, the disposable pen delivery device can be refilled with the pharmaceutical composition in the storage tank in the device. Once the pharmaceutical composition in the storage tank is used up, the entire device is discarded. Many types of repetitive pen-type and autoinjector delivery devices can be used to deliver the pharmaceutical composition of the present invention subcutaneously.

Advantageously, the above-described pharmaceutical composition for oral or parenteral use is prepared into a unit dosage form suitable for matching the dose of the active ingredient. The unit dosage form comprises, for example, tablets, pills, capsules, injections (ampoules), suppositories, and the like. The amount of the fusion protein contained in a unit dose is about 5 to about 500 mg per dosage form, especially in the case of injections; preferably, the amount of antibody should be about 5 to about 100 mg and about 10 to about 250 mg for other types of dosage forms.

The present invention also provides a method of preventing, ameliorating, or treating tumor, cancer, metastatic tumor, metastatic cancer, or infectious disease in an individual in need of prevention, amelioration, or treatment of tumor, cancer, metastatic tumor, metastatic cancer, or infectious disease, comprising administering the fusion protein of the present invention to said individual.

### Examples

The following examples describe the present invention in more detail. The examples are put forth to describe the present invention more specifically and are not intended to limit the scope of the present invention to the examples. It is evident to those skilled in the art that the examples can be modified in various ways within the scope of the claims.

The molecular biological reagents were used according to the manufacturer's instructions. Recombinant DNA techniques were performed using standard methods as described in Sambrook et al., Molecular cloning: A laboratory manual, Cold Spring Harbor Laboratory Press, Cold Spring Harbor, New York, 1991. The numbering of amino acid residues for immunoglobulin molecules or antibody molecules is indicated as the position from the N-terminus based on the EU index as in Kabat et al., Sequences of Proteins of Immunological Interest, Fifth Ed., NIH Publication, 1991.

### Example 1. Preparation of a cytokine fusion protein

### Example 1.1. Design of the structure of the fusion protein

In order to preferentially increase the activity of tumor-specific exhausted T_{eff} cells, it is important to selectively deliver cytokines that specifically induce the proliferation and/or activation of T_{eff} cells to T_{eff} cells in the tumor. To this end, we designed a structure of a cytokine fusion protein in which a cytokine capable of inducing proliferation and/or activation of T_{eff} cells and a T_{eff} cell targeting domain are connected to each other and act "in cis" on T_{eff} cells. As a cytokine capable of inducing proliferation and/or activation of T_{eff} cells, interleukin-2 (hereinafter abbreviated as IL-2 or IL2) and interleukin-15 (hereinafter abbreviated as IL-15 or IL15) were considered, which transmit signals through the βγ-subunit of an interleukin-2 receptor (IL-2Rβγ) expressed in T_{eff} cells. IL-2 was selected as an embodiment. This was linked to the Fc of an anti-PD-1 immuno-oncology antibody as a T_{eff} cell targeting domain to form a bifunctional fusion protein.

Cytokines such as IL-2, an immune protein present in the blood, are known to play an important role in intracellular signaling in various cells including immune cells. Therefore, a strategy is needed to increase the selectivity of the exogenous cytokines to act specifically only on T_{eff} cells in the tumor while reducing toxicity or side effects caused by the exogenous cytokines.

The inventors designed a fusion protein comprising IL-2 preoccupied by an α subunit of IL-2 receptor (IL-2Rα or CD25), wherein the IL-2 is connected to the C-terminus of the first Fc chain of an anti-PD-1 antibody and the IL-2Rα is connected to the C-terminus of the second Fc chain so as to induce an interaction between the IL-2 and IL-2Rα connected respectively to the ends of the first and second Fc chains of the anti-PD-1 antibody. The IL-2 and IL-2Rα proteins are linked only to the ends of the Fc chains of the anti-PD-1 antibody but are not covalently bonded to each other (see FIG. 1).

FIGs. 1 to 3 illustrate exemplary structures of the "in-cis" fusion proteins of the present invention. As shown in FIG. 2, the fusion protein may be in a structure where IL-2 and IL-2Rα are covalently bonded to each other via a linker to form a complex, and then the cytokine complex is linked to the Fc of an anti-PD-1 antibody via either IL-2 or IL-2Rα. Alternatively, as a way to solve the issues of toxicity or side effects caused by exogenous cytokines, the inventors also devised a fusion protein structure that may utilize an endogenous cytokine in which only IL-2Rα or IL-15Rα as a cytokine function domain is linked to an anti-PD-1 antibody.

Since the IL-2 is preoccupied by IL-2Rα in the structures designed by the inventors, the binding of the fusion protein to regulatory T cells (T_{reg}), Foxp3-negative CD4 T cells, some innate lymphoid cells, or other endothelial cells expressing the high-affinity IL-2 receptor (abbreviated as "IL-2Rαβγ") having all three subunits α, β (IL-2Rβ or CD122), and γ (IL-2Rγ or CD132) is weakened, whereas it binds more specifically to T_{eff} cells such as cytotoxic CD8+ T cells, memory T cells, NK-T cells, and the like expressing the intermediate-affinity IL-2 receptor (abbreviated as IL-2Rβγ) consisting of IL-2Rβ and IL-2Rγ without IL-2Rα.

### Example 1.2. Amino acid sequences of the fusion protein and construction of expression vectors

### Anti-PD-1 binding domain

Fab domain of 1G1-h70, a humanized anti-PD-1 antibody previously developed by the inventors, was used as the anti-PD-1 binding domain in this example. The amino acid sequence of the Fab domain of the anti-PD-1 1G1-h70 is as follows:
Light chain VL-CL (SEQ ID NO: 51) 
DIVMTQTPLSLSVTPGQPASISCRSSQNIVHSQGDTYLEWYLQKPGQSPQLLIYKVSKR
FSGVPDRFSGSGSGTDFTLKISRVEAEDVGVYYCFQGSHVPWTFGGGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
 variable region is underlined).
Heavy chain VH-CH1 (SEQ ID NO: 52) 
QVQLVQSGAEVKKPGASVKVSCKASGYTFTGYWMHWVRQAPGQGLEWIGMIHPNS
DTTTYNEKFKNRVTMTRDTSISTAYMELSRLRSDDTAVYYCAGTDQAAWFAFWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRV
 chain variable region is underlined).

### Linker design

In order to seek a structure where the IL-2 and IL-2α are able to maintain the interaction between them in the form of being individually connected to the end of the dimeric Fc domain chains of the anti-PD-1 immuno-oncology antibody and also able to bind to the IL-2Rβγ expressed on the cell surface, the inventors performed a crystal structure analysis and determined the appropriate lengths for the linkers connecting each of the N-terminal ends of IL-2 and IL-2Rα individually to the C-terminal ends of the Fc domain.

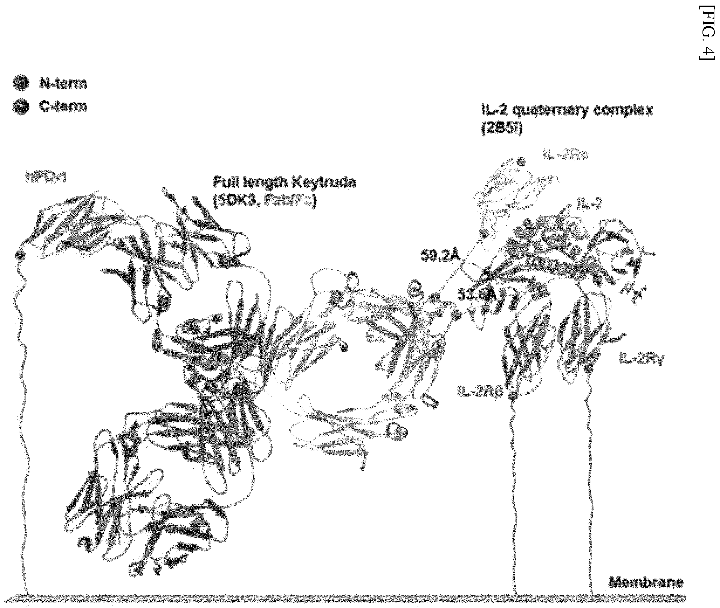

By superimposing the crystal structure of a human PD-1(hPD-1)/Fab of 1G1-h70 on the full crystal structure of Keytruda, a known anti-PD-1 antibody, the mode of binding between hPD-1 and the anti-PD-1 antibody was simulated. The C-terminal ends of the Fc domain of the anti-PD-1 antibody were placed closest to the N-terminal ends of IL-2 and IL-2Rα without colliding with the IL-2 quaternary complex, and the distances from each of the N-terminal ends of IL-2 and IL-2Rα to the corresponding C-terminal ends of the Fc domain were measured. As a result, the lengths of the connections between each of the N-terminal ends of IL-2 and IL-2Rα to the corresponding C-terminal ends of the Fc domain were determined to be 59.2 Å and 53.6 Å (FIG. 4). Through this structure-based modeling, linkers of a length suitable for linking IL-2 and IL-2Rα individually to the ends of the Fc chains of the anti-PD-1 antibody were designed.

### Improved heterodimeric Fc region

To increase the production yield of the fusion protein of the present invention, the Fc region of the anti-PD-1 antibody was prepared as a heterodimeric Fc so that the formation of a heterodimer between an Fc chain linked to IL-2 and the other Fc chain linked to IL-2Rα is favored over the formation of homodimers between the Fc chains of the same sequences. To this end, a knob-into-hole (KiH) mutation and a charged/hydrophobic amino acid mutation were introduced together in the Fc region.

The amino acid substitutions T366W and Q347R were introduced into the first Fc chain (SEQ ID NO: 49), and the amino acid substitutions T366S, L368A, Y407V and K360E were introduced into the second Fc chain (SEQ ID NO: 50). This heterodimeric Fc variant may significantly increase the production yield of the heterodimeric fusion protein to about 90% or more.

### High-affinity variant of IL-2Rα

A high-affinity variant of IL-2Rα was used to increase the binding between IL-2 and IL-2Rα. IL-2Rα L42I variant (SEQ ID NO: 5), in which leucine at position 42 of IL-2Rα is substituted with isoleucine, can bind to IL-2 with an improved affinity (KD) compared to the wild-type IL-2Rα. Table 1 below shows binding affinity and kinetics measured using SPR (surface plasmon resonance) Biacore 8K, which is for real-time observation of the interaction between proteins.

**[Table 1]**

| Ligand | Steady-state affinity K_{D} (M) | Binding kₐ (1/Ms) | k_{d} (1/s) | K_{D} (M) |
|---|---|---|---|---|
| Wild-type IL-2Rα | 2.83E-8 | 1.95E+7 | 2.16E-1 | 1.11E-8 |
| IL-2Rα L42I | 1.98E-8 | 1.08E+7 | 4.70E-2 | **4.36E-9** |

### Construction of expression vector

The expression vector was prepared using pTRIOZ_hIgG4 (S228P) (InvivoGen) expressing the human IgG4 constant region (S228P) (SEQ ID NOs: 46 and 47) so that the IL-2 C125S (SEQ ID NO: 2) or CD25 L42I (SEQ ID NO: 5) gene is inserted at the C-terminus of the Fc region. The C125S mutation was introduced into IL-2 to prevent cysteine mispairing in *E*. *coli*, as in the FDA-approved IL-2 product, Proleukin (aldesleukin). This mutation does not affect the biological activity of IL-2 (*see* Wang, A. et al., Site-specific mutagenesis of the human interleukin-2 gene: structure-function analysis of the cysteine residues, Science, 224:1431-1433, 1984). The term "wild-type IL-2" includes natural or recombinant human IL-2, IL-2 with C125S mutation, aldesleukin, as well as IL-2 of a circular permutated form, modified to have a new N-terminus and C-terminus without changing the binding properties of natural human IL-2. Non-limiting examples of the circularly permutated IL-2 include a variant wherein the existing N-terminus (Ser4) and C-terminus (Thr133) are connected to each other with a linker and a new N-terminus (Ser75) and C-terminus (Gln74) are created by cutting between them so that the IL-2 helix is arranged in the order of C-D-A-B.

Variable region sequences were inserted upstream of the constant regions of the heavy and light chains in the above expression vector together with a mouse immunoglobulin signal peptide. Basically, the vector was made according to the instructions of the vector manufacturer. The signal peptide and the antibody's VH or VL sequence were linked using overlapping PCR and placed in the pTRIOZ_hIgG4 (S228P) vector. The VH sequence was inserted after digesting the vector using the Mlu I/Nhe I restriction enzymes. The VL sequence was inserted after digesting the vector using the Asc I/BsiW I restriction enzymes.

In the first chain (knob) expression vector, the nucleotide sequence was changed to introduce T366W and Q347R mutations in the Fc region. In the second chain (hole) expression vector, the nucleotide sequence was modified using PCR to produce T366S, L368A, Y407V, and K360E mutations in the Fc region.

For the first chain (knob) expression vector, the C-terminal end was cut by the Avr II restriction enzyme; the IL-2 C125S gene with a linker of 15 amino acids was amplified using PCR techniques; and the vector was prepared using NEBuilder HiFi DNA Assembly Kit. For the second chain (hole) expression vector, the C-terminal end was cut by the Avr II restriction enzyme; the CD25 L42I gene with a linker of 20 amino acids was amplified using PCR techniques; and the vector was prepared using NEBuilder HiFi DNA Assembly Kit. The primers used are shown in the table below.

| | Primer | Sequence | Tm |
|---|---|---|---|
| IL-2 C125S | Forward | CCCTCTCCCCCGGCAAGTCAGGAGGTGGATCTGGTGGAG | 53 |
| | | (SEQ ID NO. 53) | |
| | Reverse | GCCCGAATTCGGCGGCCGCTTTATCAGGTCAGCGTAGAGATG | 54 |
| | | (SEQ ID NO. 54) | |
| CD25 L421 | Forward | Tctccctgtctccgggtaaaggaggtggaggttctggtggaggtggatctggt ggaggaggttctggtggaggtggatctGAGCTGTGTGATGATGATC (SEQ ID NO. 55) | 59.9 |
| | Reverse | gtcattggggaaacctgctcctaggTTTAGCCGGTACAGATCAG | 60.9 |
| | | (SEQ ID NO. 56) | |

The amino acid sequence coded by each expression vector is as follows:
Light Chain: VL-CL (SEQ ID NO: 51) 
DIVMTQTPLSLSVTPGQPASISCRSSQNIVHSQGDTYLEWYLQKPGQSPQLLIYKVSKR
FSGVPDRFSGSGSGTDFTLKISRVEAEDVGVYYCFQGSHVPWTFGGGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
Heavy Chain 1_Knob_with_IL2C125S: VH - CH1 *- Hinge* - CH2 - CH3 *- Linker* - **IL2C125S** (SEQ ID NO: 57) 
QVQLVQSGAEVKKPGASVKVSCKASGYTFTGYWMHWVRQAPGQGLEWIGMIHPNS
DTTTYNEKFKNRVTMTRDTSISTAYMELSRLRSDDTAVYYCAGTDQAAWFAFWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCPP
CPAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPRVYTLPPSQEEMTKNQVSLWCLVKGFYPSDIAVEWESNGQPENNYKTTPPVLD
SDGSFFLYSRLTVDKSRWQEGNVFSCSVMHEALHNHYTQKSLSLSPGKSGGGSGGGS
GGGSGGAPTSSSTKKTQLQLEHLLLDLQMILNGINNYKNPKLTRMLTFKFYMPK
KATELKHLQCLEEELKPLEEVLNLAQSKNFHLRPRDLISNINVIVLELKGSETTF
MCEYADETATIVEFLNRWITFSQSIISTLT
Heavy Chain 2_Hole_with_CD25L42I: VH - CH1 *- Hinge* - CH2 - CH3 *- Linker -* CD25L42I (SEQ ID NO: 58)

QVQLVQSGAEVKKPGASVKVSCKASGYTFTGYWMHWVRQAPGQGLEWIGMIHPNS
DTTTYNEKFKNRVTMTRDTSISTAYMELSRLRSDDTAVYYCAGTDQAAWFAFWGQ
GTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPPCPP
CPAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTENQVSLSCAVKGFYPSDIAVEWESNGQPENNYKTTPPVLDS
DGSFFLVSRLTVDKSRWQEGNVFSCSVMHEALHNHYTQKSLSLSPGKGGGGSGGGG
SGGGGSGGGGSELCDDDPPEIPHATFKAMAYKEGTMLNCECKRGFRRIKSGSIY
MLCTGNSSHSSWDNQCQCTSSATRNTTKQVTPQPEEQKERKTTEMQSPMQPVD
QASLPGHCREPPPWENEATERIYHFVVGQMVYYQCVQGYRALHRGPAESVCK
MTHGKTRWTQPQLICTG

### Example 1.3. Expression and purification of the fusion protein (Fusion Protein A)

The first chain (knob) expression vector (for expression of the first heavy chain linked to IL2C125S and the light chain) and the second chain (hole) expression vector (for expression of the second heavy chain linked to CD25L42I and the light chain) as described in Example 1.2 were co-transfected into ExpiCHO^{™} to transiently express each protein.

Transfection was performed under conditions of initial cell density of 6 x 10⁶ cells/mL and expression vector concentration of 0.8 µg DNA/mL. The ExpiCHO^{™} Expression Medium, OptiPRO^{™} SFM, and ExpiFectamine^{™} Transfection Kit (Gibco) were used following the manufacturer's instructions.

The cells that were transfected were cultured in a carbon dioxide incubator until cell viability reached 75%, after which the cell cultures were separated into cells and culture medium containing each protein by centrifugation.

Affinity chromatography using HiTrap MabselectXtra (Cytiva) column was performed to isolate the proteins of interest from the culture medium containing the recombinant proteins.

Specifically, AKTA avant25 FPLC was used for the purification process. HiTrap MabselectXtra column was equilibrated with PBS (3 mM Na₂HPO₄, 1.1 mM KH₂PO₄, 0.16 M NaCl, pH 7.2), and the culture medium collected by centrifugation was filtered through a 0.22 µm filter and then loaded onto the column. After reaction with the culture medium, the column was sequentially washed with PBS (5~10 CV) and 50 mM NaOAc pH 5.2 (5~10 CV), and the protein was eluted using the elution buffer (0.1 M Glycine pH 3.0). 3 M NaOAc pH 5.2 buffer was added to the eluted protein to make the final concentration 50~100 mM, and the eluted protein was centrifuged to remove the dye that was eluted together. The protein solution was concentrated using an Amicon tube (MWCO: 50 K), and the buffer was changed to ABS (0.1 M NaOAc, 0.15 M NaCl, pH 5.2) or PBS.

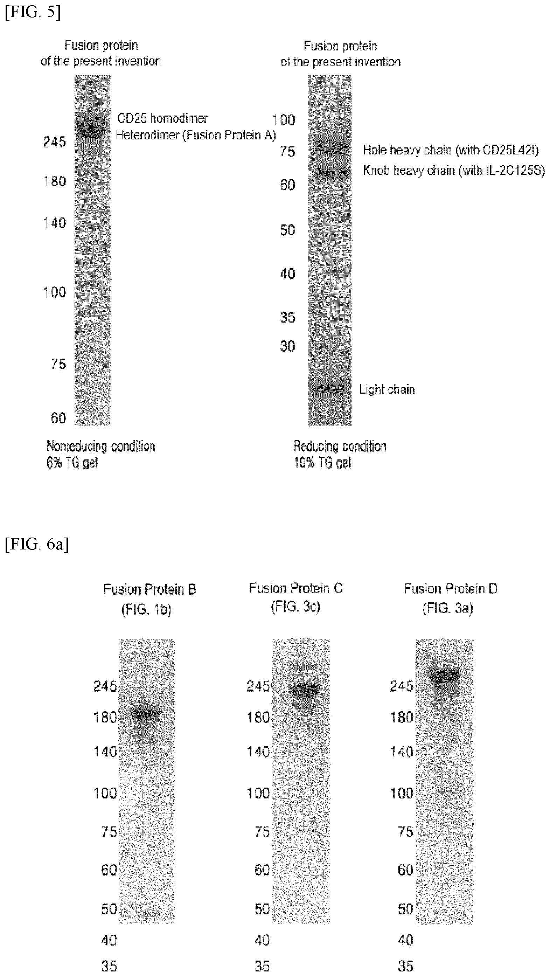

To confirm the production of the fusion protein, SDS-PAGE under non-reduction and reduction conditions was performed in 6% and 10% acrylamide-bisacrylamide (29:1) gels, respectively, and dyed with Coomassie Blue (InstantBlue Coomassie Protein Stain (Abcam, ab119211)). FIG. 5 is the SDS-PAGE results identifying the purified fusion protein (Fusion Protein A). Fusion Protein A has a heterodimeric structure where IL-2 and IL-2Rα are each individually linked to the Fc region of the anti-PD-1 antibody with two anti-PD-1 Fab arms (FIG. 1a), which can be produced in high yields.

### Example 2. Design and manufacture of various fusion proteins

### Example 2.1 Preparation of a fusion protein having one anti-PD-1 Fab arm (Fusion Protein B, FIG. 1b)

Structural design and vector construction were carried out in the same manner as in Example 1, but the expression vector of Example 1 was used as a primary vector, and the VH-CH1 part of the first heavy chain (knob) vector was removed using PCR. The amino acid sequence coded by the first heavy chain (knob) vector in Fusion Protein B is as follows.

Fc_Knob_with_IL2C125S: *Hinge* - CH2 - CH3 *- Linker* - **IL2C125S** (SEQ ID NO: 59)

KYGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEK
TISKAKGQPREPRVYTLPPSQEEMTKNQVSLWCLVKGFYPSDIAVEWESNGQPENNY
KTTPPVLDSDGSFFLYSRLTVDKSRWQEGNVFSCSVMHEALHNHYTQKSLSLSPGKS
GGGSGGGSGGGSGGAPTSSSTKKTQLQLEHLLLDLQMILNGINNYKNPKLTRMLT
FKFYMPKKATELKHLQCLEEELKPLEEVLNLAQSKNFHLRPRDLISNINVIVLEL
KGSETTFMCEYADETATIVEFLNRWITFSQSIISTLT

As the final expression vector, pG3.1 was used, which was produced in-house based on pcDNA3.1 and configured to include three MCSs (multiple cloning sites). Each MCS has the same promoter (CMV), enhancer (CMV), and Poly A signal (BGH). An expression vector was produced by individually inserting the genes of the light chain, first heavy chain (knob), and second heavy chain (hole) cloned from the primary vector to the three MCSs. The protein was expressed in the same manner as in Example 1 by transfecting the expression vector into ExpiCHO^{™} cells, and the target protein was isolated and purified in the same manner as in Example 1.

### Example 2.2 Preparation of a heterodimeric fusion protein comprising one IL-2Rα protein without IL-2 (Fusion Protein C, FIG. 3c)

Structural design, vector production, protein expression, and purification were performed in the same manner as in Example 2.1, but the IL-2 C125S gene was not connected to the C-terminal end of the first heavy chain (knob) vector. The light chain and the second heavy chain (hole) are the same as in Example 1, and the amino acid sequence of the first heavy chain (knob) is as follows.

HeavyChain 1_Knob_without_IL2C125S: VH - CH1 *- Hinge* - CH2 - CH3 (SEQ ID NO: 60)

QVQLVQSGAEVKKPGASVKVSCKASGYTFTGYWMHWVRQAPGQGLEWIGMIHPNS
DTTTYNEKFKNRVTMTRDTSISTAYMELSRLRSDDTAVYYCAGTDQAAWFAFWGQG
TTVTVSSASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPCPA
PEFLGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREP
RVYTLPPSQEEMTKNQVSLWCLVKGFYPSDIAVEWESNGQPENNYKTTPPVLDSDGS
FFLYSRLTVDKSRWQEGNVFSCSVMHEALHNHYTQKSLSLSPGK

### Example 2.3 Preparation of a homodimeric fusion protein comprising two IL-2Rα proteins without IL-2 (Fusion Protein D, FIG. 3a)

Fusion Protein D in the form of a homodimer was prepared in the same manner as in Example 1 for the structural design, expression vector production, and protein expression and purification, but the CD25 L42I (SEQ ID NO: 5) gene was connected to the C-terminal end of the Fc region without the mutations for the improvement of the heterodimer production yield. The light chain amino acid sequence of Fusion Protein D is the same as in Example 1 (SEQ ID NO: 51), and the heavy chain amino acid sequence is as follows.

HeavyChain_with_CD25L42I: VH - CH1 *- Hinge* - CH2 - CH3 *- Linker -* **CD25L42I** (SEQ ID NO: 61)

QVQLVQSGAEVKKPGASVKVSCKASGYTFTGYWMHWVRQAPGQGLEWIGMIHPNS
DTTTYNEKFKNRVTMTRDTSISTAYMELSRLRSDDTAVYYCAGTDQAAWFAFWGQG
TTVTVSSASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPCPA
PEFLGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSPGKGGGGSGGGGSGGG
GSGGGGSELCDDDPPEIPHATFKAMAYKEGTMLNCECKRGFRRIKSGSIYMLCT
GNSSHSSWDNQCQCTSSATRNTTKQVTPQPEEQKERKTTEMQSPMQPVDQASL
PGHCREPPPWENEATERIYHFVVGQMVYYQCVQGYRALHRGPAESVCKMTHG
KTRWTQPQLICTG

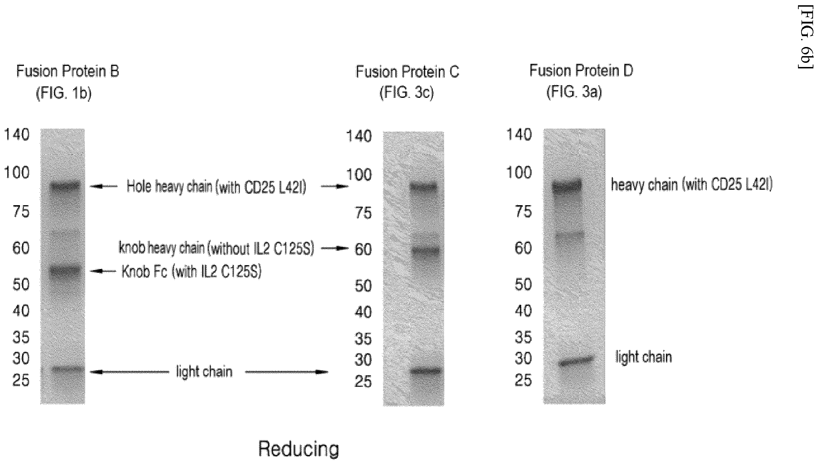

SDS-PAGE was performed in 4~15% TGX Precast gel (Biorad, BR4561086) to identify the fusion proteins prepared and purified in Examples 2.1 to 2.3, and the results are shown in FIG. 6.

### Example 3. Analysis of binding selectivity to the IL-2 receptor proteins

In order to examine the binding selectivity of the fusion protein to each IL-2 receptor due to the structure of the IL-2/IL-2Rα heterodimeric Fc region, binding of the fusion protein to IL-2Rα and IL-2Rβγ was determined by ELISA. IL-2Rα-His (Acrobiosystems) and IL-2Rβγ-His (Acrobiosystems) were added to a 96-well plate (Thermofisher scientific) at 100 µl/well, and the plate was sealed and incubated overnight at 4°C. After blocking and washing, Fusion Protein A and the control antibody (Fc-IL2; prepared in-house) were diluted to a concentration of 0 to 1000 nM, added to each well at 100 µl/well, and incubated at room temperature for 2 hours. After washing, a secondary antibody (HRP-conjugated anti-His antibody) was added and incubated at room temperature in the dark for 30 minutes. After washing, a TMB substrate solution (Abcam) was added, and the color development reaction was stopped with a STOP solution (Abcam). Absorbance at 450 nm was measured using a microplate reader (ThermoFisher).

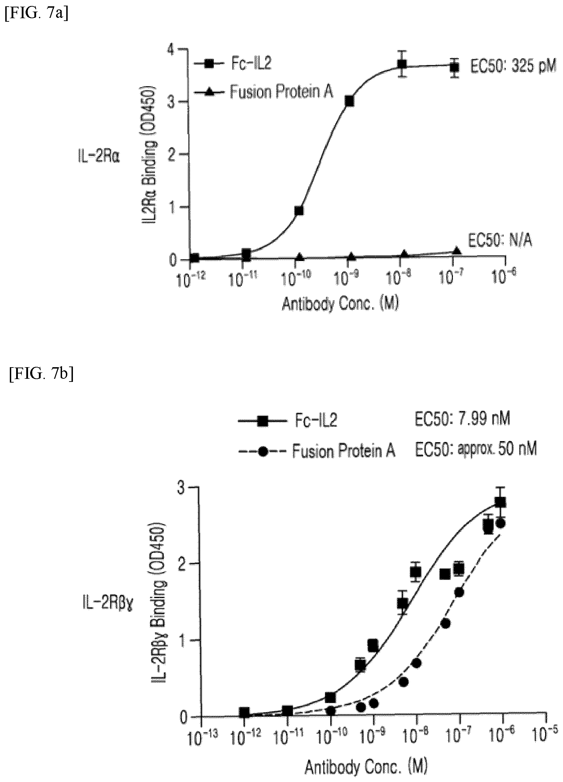

The results are shown in FIGs. 7a and 7b. Fusion Protein A did not bind to the IL-2Rα protein (FIG. 7a) but showed a weaker, yet good binding activity to the IL-2Rβγ protein, compared to the wild-type IL-2 (FIG. 7b). This shows that the fusion protein of the present invention has a selectivity for IL-2Rβγ over IL-2Rα due to its unique structure in which IL-2 is preoccupied by IL-2Rα.

### Example 4. Analysis of binding selectivity to IL-2 receptor-expressing cells depending on the presence or absence of PD-1 expression

We then examined whether the fusion protein of the present invention having IL-2/IL-2Rα fused to the anti-PD-1 binding domain may cause any change in IL-2Rβγ signaling in the exhausted PD-1⁺ T_{eff} cells existing in the tumor microenvironment (TME) due to the presence of the anti-PD-1 binding domain. HEK-Blue^{™} reporter assay system (InvivoGen) was used for this test.

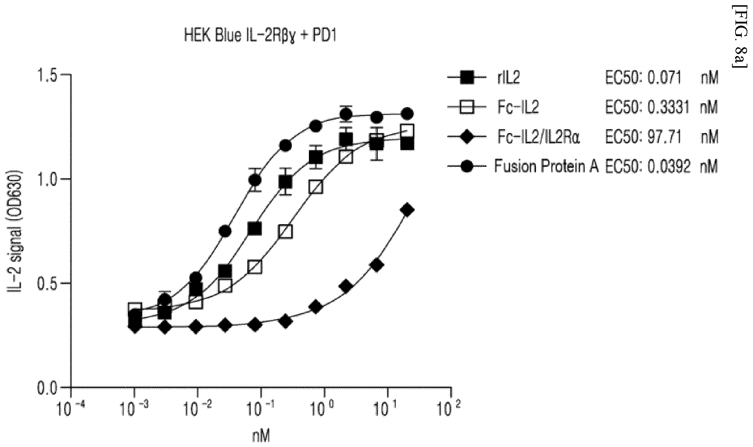

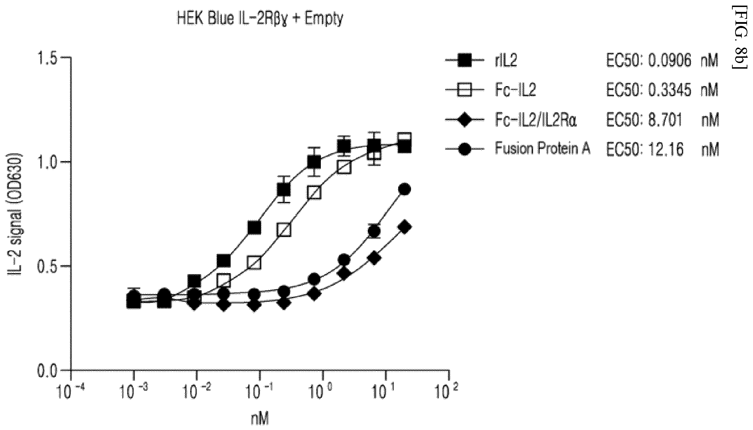

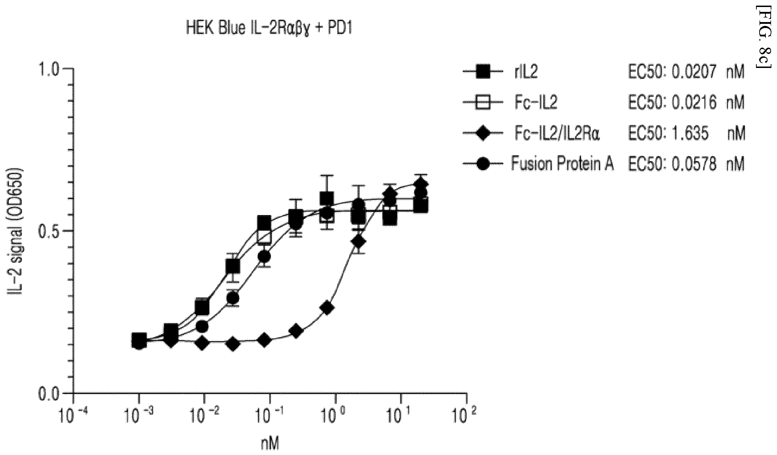

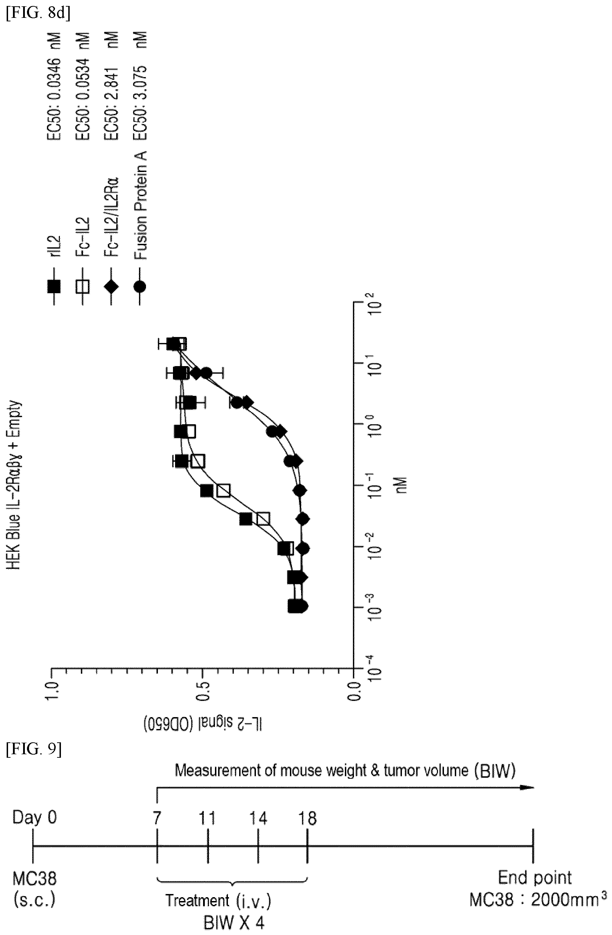

First, PD-1 expressing cells were prepared by transfecting the pCMV3-hPD1 (HG10377-CF) vector into HEK-Blue^{™} CD122/CD132 cells (InvivoGen) expressing IL-2Rβγ and HEK-Blue^{™} IL-2 cells (InvivoGen) expressing IL-2Rαβγ. As a control group, the above two cells transfected with pCMV3-Empty vector (control DNA) were used. The transfected cells were added to a 96-well plate at 50,000 cells/well and incubated in a 5% CO₂ chamber at 37°C. After removing the cell culture supernatant, Fusion Protein A, a recombinant human IL-2 (rIL2; Sigma), heterodimeric Fc-IL2 (Fc-IL2; prepared in-house), and heterodimeric Fc-IL2/IL2Rα (IL-2 and IL-2Rα are individually connected to a heterodimeric Fc, Fc-IL2/IL2Rα; prepared in-house) in 100 µl DMEM medium were added to the cells at concentrations of 0 to 20 nM and incubated at 37°C for 8 hours. The cell culture supernatant was added to a new 96-well plate at 20 µl/well, treated with QUANTI-Blue^{™} solution (InvivoGen), a colorimetric reagent, at 180 µl/well, and incubated at 37°C in the dark for about 1 hour. The activity of the secreted alkaline phosphatase (SEAP, secreted embryonic alkaline phosphatase) was measured as an OD at 620 to 655 nm with a microplate reader. The results are illustrated in FIG. 8.

The average value was obtained after repeating the above procedure twice. The EC₅₀ values of the fusion protein of the present invention depending on the presence or absence of PD-1 expression and the type of the expressed IL-2R are shown in Table 2 below.

**[Table 2]**

| EC₅₀ (nM) | | PD-1 expression | |
|---|---|---|---|
| | | PD-1 (+) | PD-1 (-) |
| IL-2Rβγ | 1st | 0.01774 | 7.274 |
| | 2nd | 0.0392 | 12.16 |
| | Mean±SD | 0.028±0.015 | 9.72±3.45 |
| IL-2Rαβγ | 1st | 0.0348 | 0.9630 |
| | 2nd | 0.0578 | 3.075 |
| | Mean±SD | 0046±0.016 | 2.02±1.49 |

As can be seen in Table 2, the fusion protein of the present invention surprisingly exerted about 340 times stronger IL-2 response in IL-2Rβγ cells expressing PD-1 than in the cells without PD-1 expression (see Table 2 and FIGs. 8a and 8b illustrating the second test results). Meanwhile, the fusion protein of the present invention exhibited a weak IL-2 response in IL-2Rβγ cells without PD-1 expression (FIG. 8b), which is thought to be due to the characteristic of "no alpha but attenuated beta gamma affinity" of the fusion protein, as shown in the binding activity test for IL-2Rβγ in Example 3 (FIG. 7b). Although not bound by the theory, it appears that such attenuated beta gamma affinity is not able to produce sufficient intracellular signaling in the cells expressing IL-2Rβγ but not expressing PD-1. However, in the cells also expressing PD-1 (e.g., PD-1⁺ T_{eff} cells in TME), the IL-2 in the fusion protein of the present invention binds "in-cis" to the cells with the help of the anti-PD-1 binding domain, thereby binding strongly to IL-2Rβγ ("switch-on" effect) to produce intracellular signaling at a significant level.

The "no alpha but attenuated beta gamma affinity" of the fusion protein of the present invention suggests that it will not bind to cells such as peripheral NK cells, which have constitutive expression of IL-2Rβγ but low PD-1 expression, and thus is advantageous compared to the conventional IL-2 proteins in terms of reducing the systemic toxicity or side effects due to NK cell overactivation.

In addition, in the cells expressing IL-2Rαβγ, the IL-2 response induced by the fusion protein of the present invention was only slightly increased by the presence of PD-1 expression, compared to the cells expressing IL-2Rβγ *(see* Table 2 and FIGs. 8c and 8d illustrating the second test results; 340-fold vs. 44-fold difference). The actual difference in the IL-2 response between the cells expressing IL-2Rαβγ and those expressing IL-2Rβγ would have been more significant, considering that cells generated to express multiple receptor subunits such as IL-2Rαβγ may have some heterogeneity in the expression of the subunits and thus may also contain some cells expressing IL-2Rβγ. This means that the fusion protein of the present invention induces a significantly low IL-2 response in T_{reg} cells that constitutively express IL-2Rαβγ or eosinophils and other endothelial cells that may promote vascular leak syndrome, and thus, it can induce an excellent anti-cancer immune response by specifically binding to the exhausted T_{eff} cells present in the TME while solving the issue of systemic toxicity or serious side effects that can be caused by the exogenous IL-2 protein.

### Example 5. Evaluation of in vivo anti-cancer efficacy in the mouse tumor model

### Example 5.1. Anti-cancer efficacy compared to anti-PD-1 or Fc-IL2/IL2Rα monotherapy

To evaluate the ability of the fusion protein of the present invention to suppress tumor growth by selectively transferring IL-2 to and activating T_{eff} cells that express PD-1 in tumors, the fusion protein was tested in the MC38 colorectal cancer syngeneic mouse tumor model for its anticancer efficacy. The experimental procedure is summarized in FIG. 9.

MC38 cancer cells were cultured in DMEM (+10% FBS, 1% streptomycin-penicillin, 1% NEAA) medium in a 5% CO₂ chamber at 37°C. MC38 cancer cells reaching passage 9 were used for transplantation in mice. 1 x 10⁶ MC38 cells were injected subcutaneously with a 1 ml insulin syringe between the middle back and the right flank (right back) of 8-week-old C57BL/6 female mice. At this time, the mice were maintained in an anesthetic state using isoflurane. Mice injected subcutaneously with MC38 cells were observed for 7 days. All mice were purchased from Orient Bio and raised in a specific-pathogen-free animal facility (SPF facility).

After 7 days, tumor size was measured, and mice with tumor size reaching 100 mm³ (±30) were separated and used for the experiment. A total of 28 mice transplanted with MC38 cells were obtained and divided into 4 groups that were treated with (1) Fusion Protein A of the present invention, (2) an antibody having the same anti-PD-1 binding domain as Fusion Protein A, (3) a heterodimeric Fc-IL2/IL2Rα, and (4) PBS as a control. Each test substance was prepared at the same dose (140 pmole) in 100 µl DPBS and then injected into the mouse tail vein twice a week (BIW) from the 7th day of MC38 cell injection for a total of 4 times (the PBS-treated group was intravenously injected with 100 µl DPBS only). From the 7th day, tumor size and body weight were measured and recorded twice a week. Mice whose tumor size reached 2000 mm³ were euthanized.

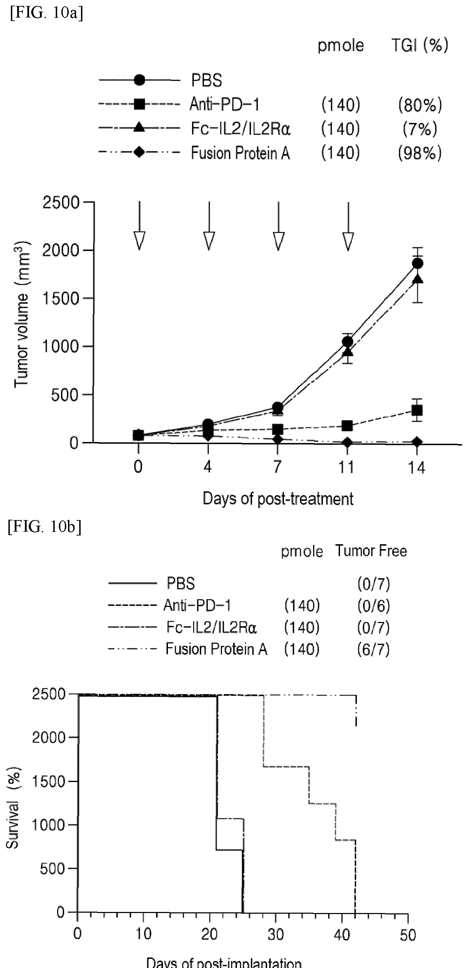

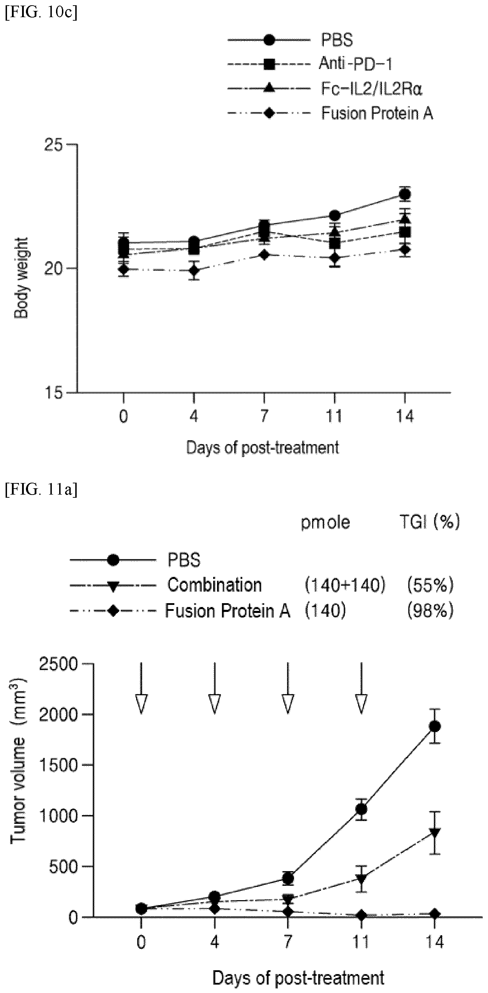

FIGs. 10a to 10c show the change in tumor size over time in each group (FIG. 10a), the survival of mice (FIG. 10b), and the change in body weight (FIG. 10c). As can be seen in FIGs. 10a and 10b, the fusion protein of the present invention was remarkably superior to the monotherapy of anti-PD-1 antibody or Fc-IL-2/IL-2Rα in tumor growth suppression and survival prolongation. The fusion protein of the present invention achieved tumor growth inhibition % (TGI) as high as 98% and maintained tumor-free state in 6 mice out of the total 7 mice. The mice did not show any reduction in body weight caused by the fusion protein of the present invention (FIG. 10c).

### Example 5.2 Anti-cancer efficacy compared to the combination therapy of anti-PD-1 and Fc-IL2/IL2Rα

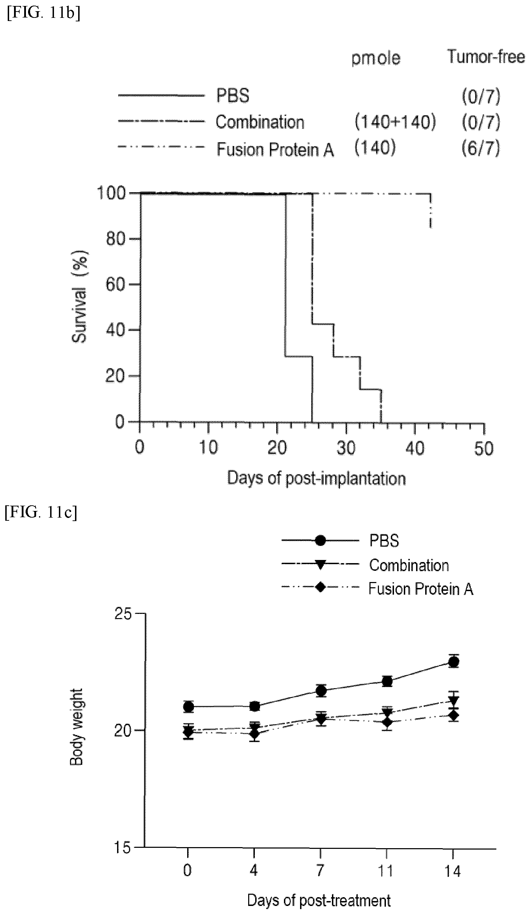

Whether the fusion protein of the present invention exhibits improved anticancer efficacy due to its unique fusion structure was evaluated using the same mouse tumor model used in Example 5.1. The experiment was performed according to the procedure described in Example 5.1, but a total of 21 mice transplanted with MC38 cells were obtained, which were divided into three groups and treated with (1) Fusion Protein A of the present invention, (2) a combination of an antibody with the same anti-PD-1 binding domain as Fusion Protein A and a heterodimeric Fc-IL2/IL2Rα, and (3) PBS as a control. The results are shown in FIG. 11.

Surprisingly, the fusion protein of the present invention had a remarkably superior effect in tumor growth inhibition and clearance, compared to the group administered with the combination of the Fc-IL2/IL2Rα and the anti-PD-1 antibody having the same anti-PD-1 binding domain as the fusion protein (FIG. 11a), and prolonged the survival of the mice significantly (FIG. 11b). These results show that the fusion protein of the present invention can effectively deliver IL-2 to T_{eff} cells expressing PD-1 in tumors and induce proliferation and activation of T_{eff} cells, thereby exhibiting excellent anti-cancer effects.

### Example 5.3 Long-term memory immune response test

To examine whether the fusion protein of the present invention can form long-term memory immunity and exhibit long-term effects even after discontinuation of administration, the inventors conducted a long-term memory immune response test with C57BL/6 mice which had remained tumor-free by administering the fusion protein of the present invention after MC38 cancer cell transplantation (the first transplantation). To this end, the procedure described in Example 5.1 was followed, but the mice were administered with 75~280 pmole of Fusion Protein A once a week (QW) or twice a week (BIW) for a total of 1 to 4 times, and a total of 26 mice that remained free of tumor (tumor-free mice) for 60 days after the first transplantation were secured and used in the experiment (75 mole/BIW/4 times: 6 mice, 140 pmole/BIW/4 times: 6 mice, 150 pmole/BIW/4 times: 5 mice, 280 pmole/BIW/4 times: 4 mice, 150 pmole/QW/once: 2 mice, 150 pmole/QW/twice: 3 mice).

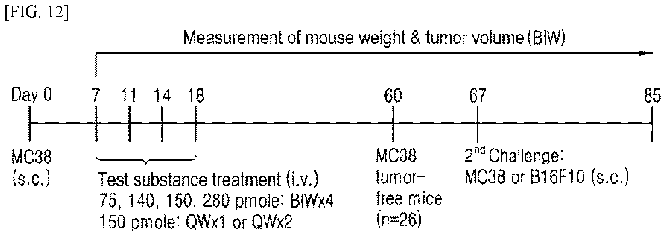

On the 67th day after the first transplantation of MC38, the above 26 mice were divided into two groups; 1 x 10⁶ MC38 cancer cells (14 mice) or B16F10 melanoma cells (12 mice) were transplanted into the right back with subcutaneous injections (the second transplantation, 2nd challenge); and tumor growth was observed. As a control group, 14 mice that did not undergo MC38 first transplantation and fusion protein administration (naïve) were divided into two groups, and MC38 or B16F10 cancer cells were transplanted in the same manner as above. Changes in tumor size were observed until the 85th day after the first transplantation. The experimental procedure is summarized in FIG. 12.

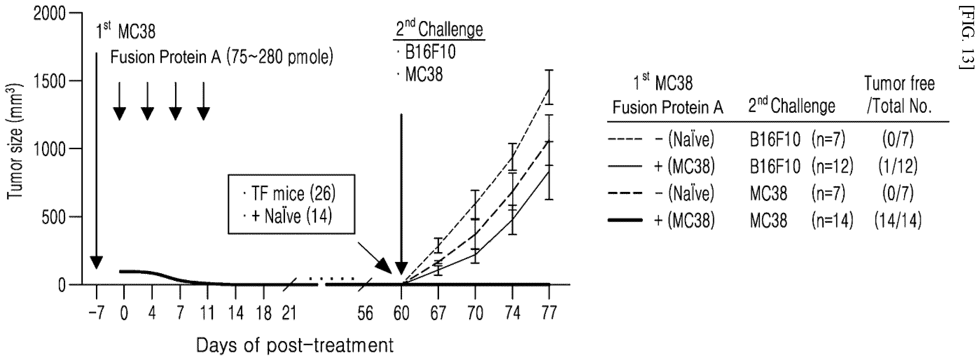

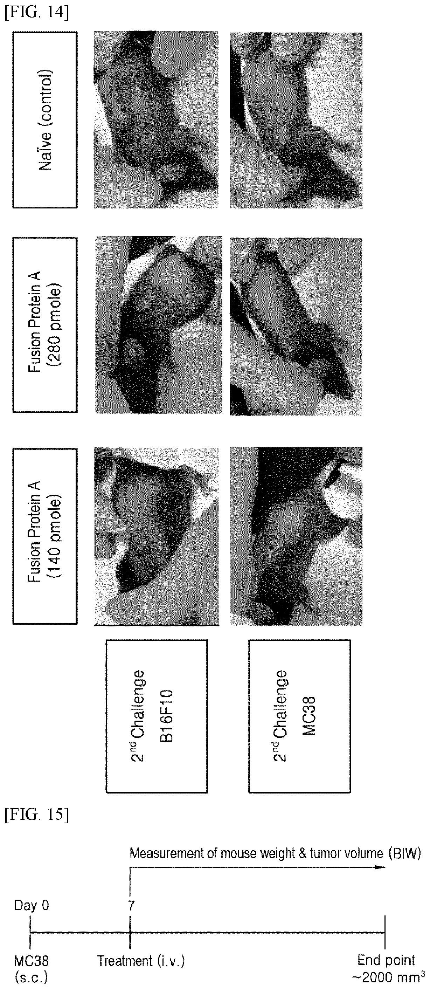

All of the 14 mice that underwent the second transplantation with MC38 cancer cells after the MC38 first transplantation and Fusion Protein A administration remained tumor-free until the end of observation, while tumor growth was observed in the other groups of mice (FIGs. 13 and 14). Among them, in the case of mice with the second transplantation of B16F10 melanoma cells, tumor growth was suppressed compared to the naive mice despite the absence of additional administration of Fusion Protein A (FIG. 13). These results show that the fusion protein of the present invention induces a tumor-specific long-term immune response to maintain an inhibitory effect on the same cancer types (acquired immune response) for a long time even after discontinuation of administration and to maintain some anti-cancer effects on other cancer types (non-tumor specific) (innate immune response).

### Example 5.4 Evaluation of dose-dependent anticancer effects upon single administration

An experiment was conducted using the MC38 colorectal cancer syngeneic mouse tumor model to determine whether the anticancer effect of the fusion protein of the present invention changes in a dose-dependent manner.

The experiment was conducted in the same manner as in Example 5.1, but on the 7th day after the transplantation, mice with a tumor size of 100 mm³±30 were divided into a total of 7 groups (5 or 6 mice per group), and Fusion Protein A was administered once to each group at a dose of 0 (PBS), 50, 150, 300, 600, 1200, or 3000 pmole. Tumor size and body weight were measured and recorded twice a week. When the tumor size reached 2000 mm³, the mice were euthanized. The experimental procedure is summarized in FIG. 15.

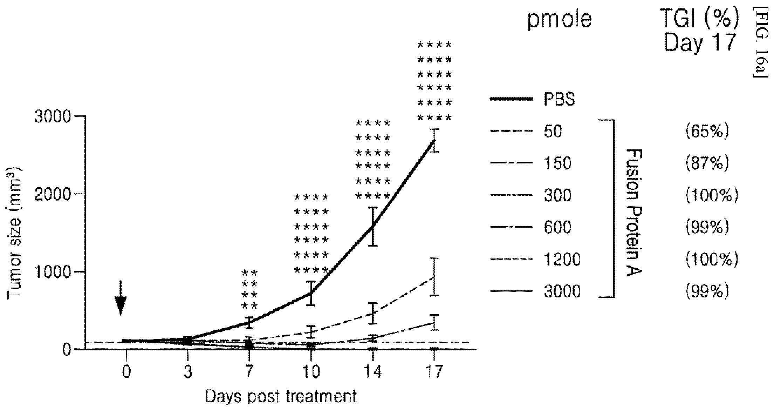

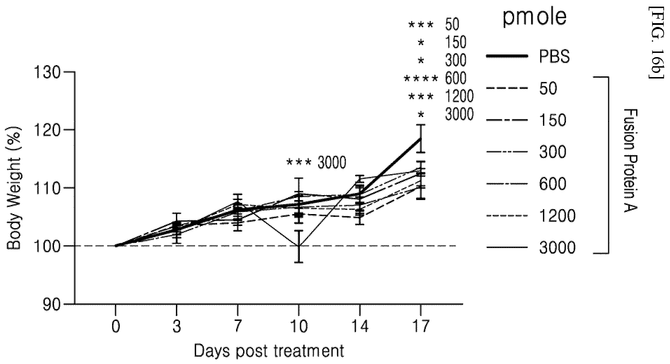

As shown in FIG. 16a, Fusion Protein A of the present invention showed an increased tumor suppression effect in proportion to the dose in the range of 0 to 150 pmole, and the TGI reached 99~100% at all doses of 300 pmole or higher. Therefore, it is judged that the dose in which the anti-cancer effect against MC38 is saturated is 150 to 300 pmole upon a single administration. In terms of weight change, weight loss was seen at 3000 pmole on the 10th day after administration. The weight was subsequently recovered, and no weight loss was observed at all other doses (FIG. 16b) (in the figure, * p<0.05, ** p<0.01, *** p<0.001, **** p<0.0001; two-way analysis of variance (two-way ANOVA) with a Tukey post-test for multiple comparison (to compare each group), compared to PBS control group).

### Example 5.5 Evaluation of anticancer effects in B16F10 melanoma syngeneic model

The experiment was conducted in the same manner as in Example 5.1, but the efficacy of the fusion protein was evaluated in 9-week-old C57BL/6J mice transplanted with 7.5x10⁵ B16F10 cells instead of MC38 cancer cells.

On the seventh day of transplantation, when the tumor size reached an average of 96 mm³, the mice were divided into three groups, and each group was administered once with PBS (10 mice), Fusion Protein A 300 pmole (5 mice), or Fusion Protein A 600 pmole (5 mice). Tumor size and body weight were measured and recorded twice a week.

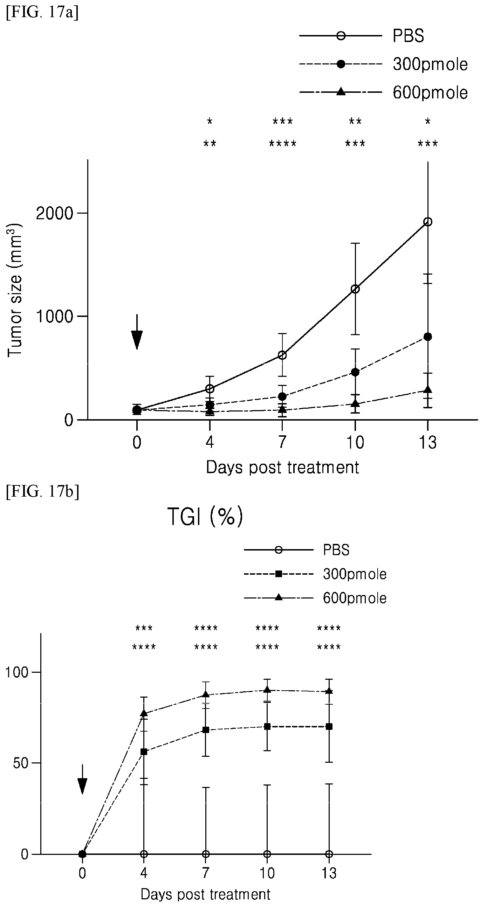

FIG. 17 shows a change in tumor size (FIG. 17a) and tumor growth inhibition rate (TGI%) (FIG. 17b) over time in each group. As shown in FIGs. 17a and 17b, the groups treated with Fusion Protein A showed statistically significant tumor growth inhibition compared to the PBS-treated control group (TGI 70.2% (300 pmole) and 89.2% (600 pmole) compared to PBS-treated group as of the 13th day after administration; in the figure, * p<0.05, ** p<0.01, *** p<0.001, **** p<0.0001, two-way analysis of variance with a Tukey post-test).

### Example 5.6 Evaluation of anticancer effects in Pan02 pancreatic cancer syngeneic model

The experiment was conducted in the same manner as in Example 5.1, but the efficacy of the fusion protein was evaluated in mice in which 3x10⁶ Pan02 cancer cells were transplanted instead of MC38 cancer cells.

On the 7^{th} day of the transplantation, when the tumor size reached an average of 71 mm³, the mice were divided into two groups: the Fusion Protein A treatment group (5 mice) and the PBS treatment group (5 mice). Fusion Protein A was mixed with 100 µl DPBS and injected into the mouse tail vein at 75 pmole per mouse twice a week (BIW) for a total of 4 times (for the PBS treatment group, only 100 µl DPBS was intravenously injected). Tumor size and body weight were measured and recorded twice a week from the 7th day to the 40th day of transplantation.

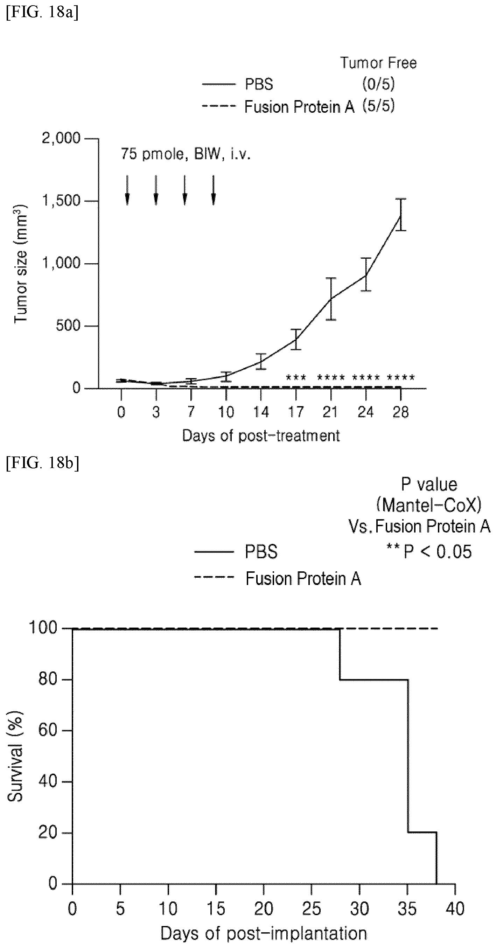

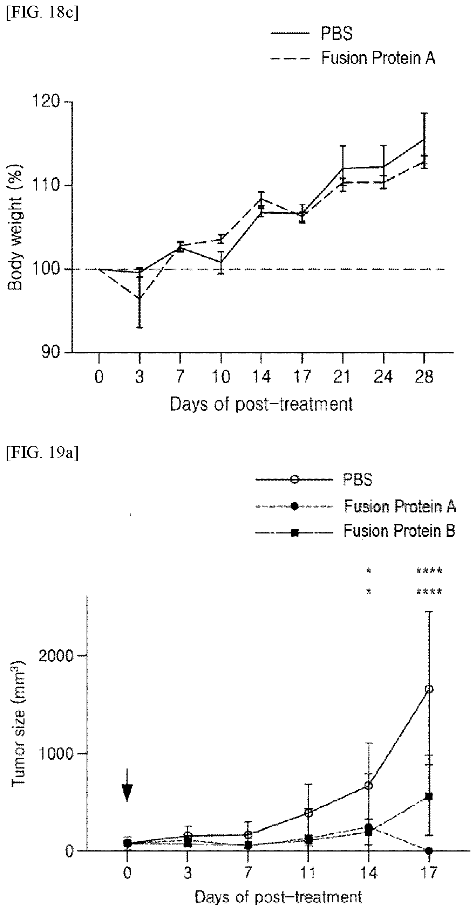

FIG. 18 shows the change in tumor size (FIG. 18a), mouse survival period (FIG. 18b), and mouse weight change (FIG. 18c) over time in each group. Fusion Protein A showed excellent anti-tumor activity maintaining a tumor-free state in all of the 5 mice starting from the third administration until the end of observation (FIG. 18a; TGI 100%, Day 17 after administration: *** p<0.001, Days 21-28 after administration: **** p<0.0001; two-way analysis of variance with a Tukey post-test, compared to the PBS control group), and all mice survived until the end of the observation (FIG. 18b). In terms of weight change, weight loss was temporarily observed after the first administration of Fusion Protein A, but it was recovered, and no further weight loss was observed (FIG. 18c).

### Example 5.7 Comparative evaluation of anticancer effects according to the number of anti-PD-1 Fab arms

An experiment was conducted using the MC38 colorectal cancer syngeneic mouse tumor model to determine how the anti-cancer effect of the fusion protein of the present invention changes depending on the number of anti-PD-1 Fab arms. The experiment was conducted in the same manner as in Example 5.1, but on the 7th day after MC38 cancer cells were transplanted, either Fusion Protein A (two PD-1 Fab arms) or Fusion Protein B (one PD-1 Fab arm) was administered once at a dose of 450 pmole (5 mice per group), and PBS was administered to 7 mice as a control group.

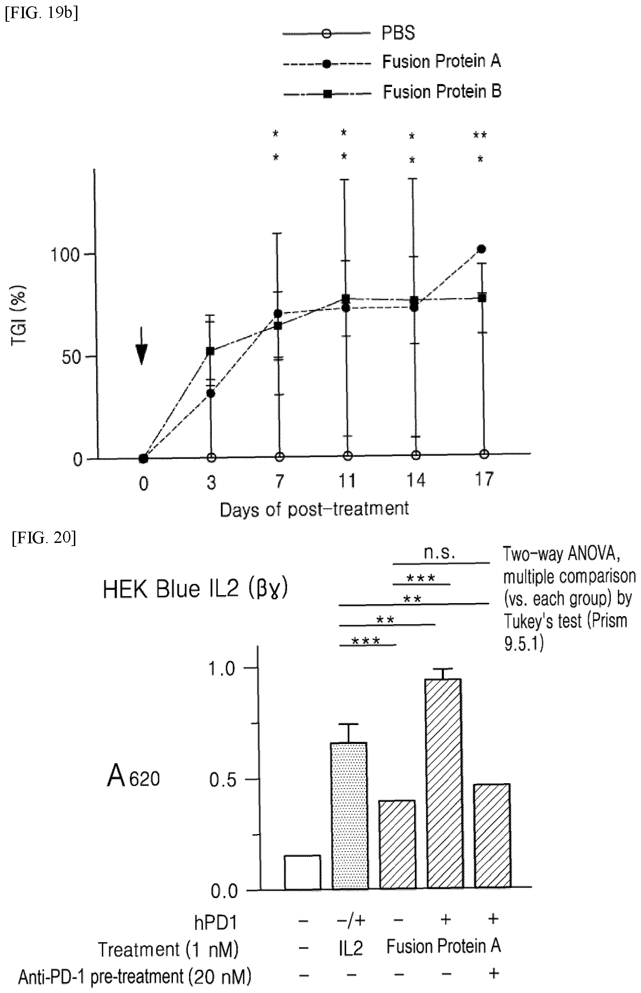

Tumor size and body weight were measured and recorded twice a week until the 17th day after administration. FIG. 19 shows the change in tumor size (FIG. 19a) and tumor growth inhibition rate (TGI%) (FIG. 19b) over time in each group. As shown in FIGs 19a and 19b, the Fusion Protein A treatment group and the Fusion Protein B treatment group showed statistically significant tumor growth inhibition effects compared to the PBS treatment control group (TGI 100% (Fusion Protein A) and 76% (Fusion Protein B) compared to the PBS treatment group as of the 17th day after administration; in the figures, * p<0.05, ** p<0.01, *** p<0.001, **** p<0.0001, two-way analysis of variance with Dunnett post-test).

### Example 6. Comparative evaluation of antigen binding activities according to pH

It is known that there is a pH difference between blood (approximately pH 7.4) and the tumor microenvironment (approximately pH 5.6 to 6.8). Therefore, the binding ability at weak acidic pH in the tumor microenvironment has an important effect on the therapeutic efficacy of immuno-oncology antibodies. In previous studies by these inventors, 1G1-h70, which is used as an anti-PD-1 binding domain in the fusion protein of the present invention, had been found to have a much stronger binding affinity for human PD-1 than Keytruda and Opdivo, representative anti-PD-1 antibody drugs, at a low pH (6.0) in the tumor microenvironment (PCT/KR2022/001714). Therefore, in order to confirm whether Fusion Protein A of the present invention maintains excellent binding affinity in the tumor microenvironment, binding kinetics for human PD-1 was measured by SPR (Biacore 8K, Cytiva) analysis. As a control group for comparison, anti-human PD-1 antibodies, Keytruda (MSD) and Opdivo (BMS), were purchased from Shinwon Pharmacy Co., Ltd. as products for human use.

Human PD-1 (Acrobiosystems, Cat#PD1-H5221) at a concentration of 1 µg/ml was immobilized on a CM5 chip (Cytiva) using an amine coupling kit (Cytiva) according to the manufacturer's instructions. Seven concentrations (0-100 nM) of the control antibodies (Keytruda, Opdivo), 1G1-h70, and Fusion Protein A were flowed over the CM5 chip at a flow rate of 30 µl/min for an association phase of 180 seconds, which was followed by a dissociation phase of 1200 seconds. After each experiment in each concentration cycle, the chip was regenerated with glycine at pH 1.5. At this time, the running buffer used was a pH 7.4 or pH 6.0 HBS-EP+ buffer (0.1 M HEPES, 1.5 M NaCl, 0.03 M EDTA and 0.5% v/v Surfactant P20), and the experimental proteins were diluted in the running buffer.

The analysis was performed using the 1: 1 binding model in Biacore evaluation software version 3.0, and the results of the kinetic constants (ka and kd) and affinity (KD) values are shown in Table 3 below.

**[Table 3]**

| | pH 7.4 | | | pH 6.0 | | |
|---|---|---|---|---|---|---|
| | ka (1/Ms) | kd (1/s) | KD (M) | ka (1/Ms) | kd (1/s) | KD (M) |
| Keytruda | 7.43E+5 | 3.61E-4 | 4.86E-10 | 1.04E+6 | 7.75E-4 | 7.42E-10 |
| Opdivo | 5.63E+5 | 3.65E-4 | 6.48E-10 | 6.60E+5 | 5.12E-4 | 7.75E-10 |
| 1G1-h70 | 1.48E+5 | 3.51E-5 | 2.37E-10 | 2.86E+5 | 1.38E-5 | **4.81E-11** |
| Fusion Protein A | 1.37E+5 | 7.27E-5 | 5.30E-10 | 4.86E+5 | 2.31E-5 | **4.76E-11** |

As shown in Table 3, Fusion Protein A of the present invention and 1G1-h70 antibody showed a similar level of binding affinity (KD) to those of Keytruda and Opdivo at pH 7.4 (blood), but at pH 6.0 (tumor microenvironment), they showed about 15 to 16 times stronger binding affinity than Keytruda and Opdivo. Therefore, it has been confirmed that the fusion protein of the present invention maintains the excellent binding affinity of the anti-PD-1 antibody 1G1-h70 at the low pH of the tumor microenvironment.

### Example 7. Analysis on PD-1-mediated IL-2 signal transduction

To verify whether the fusion protein of the present invention induces PD-1-mediated IL-2 signaling, IL-2 signaling activity according to the presence or absence of pre-treatment with an anti-PD-1 antibody was analyzed using the HEK-Blue^{™} reporter assay system (InvivoGen).

The pCMV3-hPD1 vector or pCMV3-Empty vector (control) was transfected into HEK-Blue^{™} CD122/CD132 cells expressing IL-2Rβγ (InvivoGen). The transfected cells were added into a 96 well plate at a concentration of approximately 50,000 cells/well and cultured at 37°C in a 5% CO₂ chamber. After removing the cell culture supernatant, 1 µM of an anti-PD-1 antibody (1G1-h70 antibody used in Example 6) or a control isotype (human IgG4) antibody was added 20 µL per well and incubated for 40 minutes. Thereafter, recombinant human IL-2 (rhIL-2) or Fusion Protein A at a concentration of 1 nM was treated and cultured at 37°C for 8 hours. After adding the cell culture supernatant to a new 96 well plate at 20 µl/well, it was treated with a colorimetric analysis reagent QUANTI-Blue^{™} solution (InvivoGen) at 180 µl/well and incubated in the dark for at least about an hour in a 37°C incubator. OD was measured at 620 nm using a microplate reader at 30 minute intervals after starting of the reaction. The result is shown in FIG. 20.

As shown in FIG. 20, the fusion protein of the present invention exhibited a weaker signaling response than IL-2 in cells that do not express PD-1 (PD-1⁻CD122⁺CD132⁺ cells) (attenuated IL-2Rβγ binding characteristics), whereas it exhibited a much stronger response than IL-2 in cells that express PD-1 (PD-1⁺CD122⁺CD132⁺ cells). This suggests that the fusion protein of the present invention acts on PD-1 expressing cells specifically and selectively. In addition, the IL-2 response of the fusion protein of the present invention was completely neutralized when the PD-1 on the cells had been pre-blocked by pre-treatment with the anti-PD-1 antibody before the treatment with the fusion protein of this invention. This suggests that the IL-2 signaling induced by the fusion protein of the present invention is mediated by PD-1.

Exemplary embodiments have been described in detail for a clear understanding of this invention as above. However, the descriptions and examples above should not be construed as limiting the scope of this invention. The scope of the present invention is defined by the appended claims and their equivalents. The entire disclosure of all patents and scientific literature cited herein is incorporated herein by reference.

### Sequence Listing

**[Table 4]**

| **SEQ ID NO** | Description | Sequence |
|---|---|---|
| 1 | Human IL-2 (UniProt P60568 position 21-153) | |
| 2 | Human IL-2 (C125S) | APTSSSTKKTQLQLEHLLLDLQMILNGINNYKNPKLTR MLTFKFYMPKKATELKHLQCLEEELKPLEEVLNLAQSK NFHLRPRDLISNINVIVLELKGSETTFMCEYADETATIVE FLNRWITFSQSIISTLT |
| 3 | Human IL-2Rα (UniProt P01589 position 22-272) | ELCDDDPPEIPHATFKAMAYKEGTMLNCECKRGFRRIK SGSLYMLCTGNSSHSSWDNQCQCTSSATRNTTKQVTPQ PEEQKERKTTEMQSPMQPVDQASLPGHCREPPPWENE ATERIYHFVVGQMVYYQCVQGYRALHRGPAESVCKMT HGKTRWTQPQLICTGEMETSQFPGEEKPQASPEGRPESE TSCLVTTTDFQIQTEMAATMETSIFTTEYQVAVAGCVFL LISVLLLSGLTWQRRQRKSRRTI |
| 4 | Human IL-2Rα extracellular domain (UniProt P01589 position 22-240) | ELCDDDPPEIPHATFKAMAYKEGTMLNCECKRGFRRIK SGSLYMLCTGNSSHSSWDNQCQCTSSATRNTTKQVTPQ PEEQKERKTTEMQSPMQPVDQASLPGHCREPPPWENE ATERIYHFVVGQMVYYQCVQGYRALHRGPAESVCKMT HGKTRWTQPQLICTGEMETSQFPGEEKPQASPEGRPESE TSCLVTTTDFQIQTEMAATMETSIFTTEYQ |
| 5 | Human IL-2Rα mutant (UniProt P01589 position 22-186, L42I) | ELCDDDPPEIPHATFKAMAYKEGTMLNCECKRGFRRIK SGSIYMLCTGNSSHSSWDNQCQCTSSATRNTTKQVTPQ PEEQKERKTTEMQSPMQPVDQASLPGHCREPPPWENE ATERIYHFVVGQMVYYQCVQGYRALHRGPAESVCKMT HGKTRWTQPQLICTG |
| 6 | Human IL-15 (UniProt P40933 position 49-162) | NWVNVISDLKKIEDLIQSMHIDATLYTESDVHPSCKVTA |
| | | |
| 7 | Human IL-15Rα (UniProt Q13261 position 31-267) | ITCPPPMSVEHADIWVKSYSLYSRERYICNSGFKRKAGT SSLTECVLNKATNVAHWTTPSLKCIRDPALVHQRPAPPS TVTTAGVTPQPESLSPSGKEPAASSPSSNNTAATTAAIVP GSQLMPSKSPSTGTTEISSHESSHGTPSQTTAKNWELTAS ASHQPPGVYPQGHSDTTVAISTSTVLLCGLSAVSLLACY LKSRQTPPLASVEMEAMEALPVTWGTSSRDEDLENCS HHL |
| 8 | Human IL-15Rα extracellular domain (UniProt Q13261 position 31-205) | ITCPPPMSVEHADIWVKSYSLYSRERYICNSGFKRKAGT SSLTECVLNKATNVAHWTTPSLKCIRDPALVHQRPAPPS TVTTAGVTPQPESLSPSGKEPAASSPSSNNTAATTAAIVP GSQLMPSKSPSTGTTEISSHESSHGTPSQTTAKNWELTAS ASHQPPGVYPQGHSDTT |
| 9 | HCDR1 amino acids | GYWMH |
| 10 | HCDR2 amino acids | MIHPNSDTTTYNEKFKN |
| 11 | HCDR2 amino acids | MIHPNSDTTVYNEKFKN |
| 12 | HCDR2 amino acids | MIHPNSDTTIYNEKFKN |
| 13 | HCDR2 amino acids | MIHPNSDTTTYNWKFKN |
| 14 | HCDR2 amino acids | MIHPNSDTTVYNWKFKN |
| 15 | HCDR2 amino acids | MIHPNSDTTIYNWKFKN |
| 16 | HCDR3 amino acids | TDQAAWFAF |
| 17 | HCDR3 amino acids | TDQEAWFAF |
| 18 | HCDR3 amino acids | TDQAAWAAF |
| 19 | HCDR3 amino acids | TDQAAWMAF |
| 20 | HCDR3 amino acids | TDQAAWHAF |
| 21 | HCDR3 amino acids | TDQAAWFGF |
| 22 | HCDR3 amino acids | TDQEAWAAF |
| 23 | HCDR3 amino acids | TDQEAWMAF |
| 24 | HCDR3 amino acids | TDQEAWHAF |
| 25 | HCDR3 amino acids | TDQEAWFGF |
| 26 | HCDR3 amino acids | TDQAAWAGF |
| 27 | HCDR3 amino acids | TDQAAWMGF |
| 28 | HCDR3 amino acids | TDQAAWHGF |
| 29 | HCDR3 amino acids | TDQEAWAGF |
| 30 | HCDR3 amino acids | TDQEAWMGF |
| 31 | HCDR3 amino acids | TDQEAWHGF |
| 32 | LCDR1 amino acids | RSSQNIVHSNGDTYLE |
| 33 | LCDR1 amino acids | RSSQNIVHSQGDTYLE |
| 34 | LCDR1 amino acids | RSSQNIVRSNGDTYLE |
| 35 | LCDR1 amino acids | RSSQNIVRSQGDTYLE |
| 36 | LCDR2 amino acids | KVSKRFS |
| 37 | LCDR3 amino acids | FQGSHVPWT |
| 38 | HCVR amino acids | EVQLQQSGAELVKPGASVKLSCKASGYTFTGYWMH WVKQRPGQGLEWIGMIHPNSDTTTYNEKFKNRATLTV DKSSGTAYMQLSSLTSEDSAVYYCTGTDQAAWFAFWG QGTLVTVSA |
| 39 | LCVR amino acids | DIVLTQTPLSLPVSLGDQASISCRSSQNIVHSNGDTYLE WYLQKPGQSPKLLIYKVSKRFSGVPDRFSGSGSGTDFT LKISRVEAEDLGVYYCFQGSHVPWTFGGGTKLEIK |
| 40 | Humanized antibody, 1G1-h61 VH amino acids | QVQLVQSGAEVKKPGASVKVSCKASGYTFTGYWMHW VRQAPGQGLEWMGMIHPNSDTTTYNEKFKNRVTMTR DTSISTAYMELSRLRSDDTAVYYCAGTDQAAWFAFWG QGTTVTVSS |
| 41 | Humanized antibody, 1G1-h61 VL amino acids | DIVMTQTPLSLSVTPGQPASISCRSSQNIVHSNGDTYLE WYLQKPGQSPKLLIYKVSKRFSGVPDRFSGSGSGTDFT LKISRVEAEDVGVYYCFQGSHVPWTFGGGTKVEIK |
| 42 | Humanized antibody, 1G1-h68 VH amino acids | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCTGTDQAAWFAFWGQG |
| | | TTVTVSS |
| 43 | Humanized antibody, 1G1-h68 VL amino acids | DIVMTQTPLSLSVTPGQPASISCRSSQNIVHSNGDTYLE WYLQKPGQSPQLLIYKVSKRFSGVPDRFSGSGSGTDFT LKISRVEAEDVGVYYCFQGSHVPWTFGGGTKVEIK |
| 44 | Humanized antibody, 1G1-h70 VH amino acids | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSS |
| 45 | Humanized antibody, 1G1-h70 VL amino acids | DIVMTQTPLSLSVTPGQPASISCRSSQNIVHSQGDTYLE WYLQKPGQSPQLLIYKVSKRFSGVPDRFSGSGSGTDFT LKISRVEAEDVGVYYCFQGSHVPWTFGGGTKVEIK |
| 46 | Human IgG4(S228P) constant region, heavy chain amino acids | ASTKGPSVFPLAPCSRSTSESTAALGCLVKDYFPEPVTV SWNSGALTSGVHTFPAVLQSSGLYSLSSVVTVPSSSLGT KTYTCNVDHKPSNTKVDKRVESKYGPPCPPCPAPEFLG GPSVFLFPPKPKDTLMISRTPEVTCVVVDVSQEDPEVQF NWYVDGVEVHNAKTKPREEQFNSTYRVVSVLTVLHQD WLNGKEYKCKVSNKGLPSSIEKTISKAKGQPREPQVYT LPPSQEEMTKNQVSLTCLVKGFYPSDIAVEWESNGQPE NNYKTTPPVLDSDGSFFLYSRLTVDKSRWQEGNVFSCS VMHEALHNHYTQKSLSLSPGK |
| 47 | Human IgG4 constant region, kappa chain amino acids | |
| 48 | Human PD-1 protein, full amino acids | MQIPQAPWPVVWAVLQLGWRPGWFLDSPDRPWNPPTFS PALLVVTEGDNATFTCSFSNTSESFVLNWYRMSPSNQT DKLAAFPEDRSQPGQDCRFRVTQLPNGRDFHMSVVRA RRNDSGTYLCGAISLAPKAQIKESLRAELRVTERRAEVP TAHPSPSPRPAGQFQTLVVGVVGGLLGSLVLLVWVLAVI CSRAARGTIGARRTGQPLKEDPSAVPVFSVDYGELDFQ WREKTPEPPVPCVPEQTEYATIVFPSGMGTSSPARRGSA DGPRSAQPLRPEDGHCSWPL (The signal peptide is indicated in italics, and the extracellular domain is underlined) |
| 49 | Fc mutant chain 1 IgG4 216~447 S228P/Q347R/ T366W | ESKYGPPCPPCPAPEFLGGPSVFLFPPKPKDTLMISRTPE VTCVVVDVSQEDPEVQFNWYVDGVEVHNAKTKPREE QFNSTYRVVSVLTVLHQDWLNGKEYKCKVSNKGLPSSI EKTISKAKGQPREPRVYTLPPSQEEMTKNQVSLWCLVK GFYPSDIAVEWESNGQPENNYKTTPPVLDSDGSFFLYSR LTVDKSRWQEGNVFSCSVMHEALHNHYTQKSLSLSPG K |
| 50 | Fc mutant chain 2 IgG4 216~447 S228P/ K360E/ T366S/ L368A/Y407V | ESKYGPPCPPCPAPEFLGGPSVFLFPPKPKDTLMISRTPE VTCVVVDVSQEDPEVQFNWYVDGVEVHNAKTKPREE QFNSTYRVVSVLTVLHQDWLNGKEYKCKVSNKGLPSSI EKTISKAKGQPREPQVYTLPPSQEEMTENQVSLSCAVK |
| | | GFYPSDIAVEWESNGQPENNYKTTPPVLDSDGSFFLVSR LTVDKSRWQEGNVFSCSVMHEALHNHYTQKSLSLSPG K |
| 51 | Humanized anti-PD-1, VL-CL amino acids | DIVMTQTPLSLSVTPGQPASISCRSSQNIVHSQGDTYLE WYLQKPGQSPQLLIYKVSKRFSGVPDRFSGSGSGTDFT LKISRVEAEDVGVYYCFQGSHVPWTFGGGTKVEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC (The variable region is underlined) |
| 52 | Humanized anti-PD-1, VH-CH1 amino acids | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSSASTKGPSVFPLAPCSRSTSESTAALGCLVKD YFPEPVTVSWNSGALTSGVHTFPAVLQSSGLYSLSSVVT VPSSSLGTKTYTCNVDHKPSNTKVDKRV (The variable region is underlined) |
| 53 | Forward primer | CCCTCTCCCCCGGCAAGTCAGGAGGTGGATCTGGTG GAG |
| 54 | Reverse Primer | GCCCGAATTCGGCGGCCGCTTTATCAGGTCAGCGTAG AGATG |
| 55 | Forward primer | Tctccctgtctccgggtaaaggaggtggaggttctggtggaggtggatctggtggag gaggttctggtggaggtggatctGAGCTGTGTGATGATGATC |
| 56 | Reverse Primer | gtcattggggaaacctgctcctaggTTTAGCCGGTACAGATCAG |
| 57 | Humanized anti-PD-1, IgG4 heavy chain 1, knob Fc with IL-2 C125S | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSSASTKGPSVFPLAPCSRSTSESTAALGCLVKD YFPEPVTVSWNSGALTSGVHTFPAVLQSSGLYSLSSVVT VPSSSLGTKTYTCNVDHKPSNTKVDKRVESKYGPPCPP CPAPEFLGGPSVFLFPPKPKDTLMISRTPEVTCVVVDVS QEDPEVQFNWYVDGVEVHNAKTKPREEQFNSTYRVVS VLTVLHQDWLNGKEYKCKVSNKGLPSSIEKTISKAKGQ PREPRVYTLPPSQEEMTKNQVSLWCLVKGFYPSDIAVE WESNGQPENNYKTTPPVLDSDGSFFLYSRLTVDKSRWQ EGNVFSCSVMHEALHNHYTQKSLSLSPGKSGGGSGGGS GGGSGGAPTSSSTKKTQLQLEHLLLDLQMILNGINN YKNPKLTRMLTFKFYMPKKATELKHLQCLEEELKP LEEVLNLAQSKNFHLRPRDLISNINVIVLELKGSETT FMCEYADETATIVEFLNRWITFSQSIISTLT |
| 58 | Humanized anti-PD-1, IgG4 Heavy chain 2, hole Fc with CD25 L42I | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSSASTKGPSVFPLAPCSRSTSESTAALGCLVKD YFPEPVTVSWNSGALTSGVHTFPAVLQSSGLYSLSSVVT VPSSSLGTKTYTCNVDHKPSNTKVDKRVESKYGPPCPP CPAPEFLGGPSVFLFPPKPKDTLMISRTPEVTCVVVDVS |
| | | QEDPEVQFNWYVDGVEVHNAKTKPREEQFNSTYRVVS VLTVLHQDWLNGKEYKCKVSNKGLPSSIEKTISKAKGQ PREPQVYTLPPSQEEMTENQVSLSCAVKGFYPSDIAVE WESNGQPENNYKTTPPVLDSDGSFFLVSRLTVDKSRWQ EGNVFSCSVMHEALHNHYTQKSLSLSPGKGGGGSGGG GSGGGGSGGGGSELCDDDPPEIPHATFKAMAYKEGT MLNCECKRGFRRIKSGSIYMLCTGNSSHSSWDNQC QCTSSATRNTTKQVTPQPEEQKERKTTEMQSPMQP VDQASLPGHCREPPPWENEATERIYHFVVGQMVYY QCVQGYRALHRGPAESVCKMTHGKTRWTQPQLIC TG |
| 59 | Fc_Knob_with_I L-2C125S | KYGPPCPPCPAPEFLGGPSVFLFPPKPKDTLMISRTPEVT CVVVDVSQEDPEVQFNWYVDGVEVHNAKTKPREEQF NSTYRVVSVLTVLHQDWLNGKEYKCKVSNKGLPSSIEK TISKAKGQPREPRVYTLPPSQEEMTKNQVSLWCLVKGF YPSDIAVEWESNGQPENNYKTTPPVLDSDGSFFLYSRLT VDKSRWQEGNVFSCSVMHEALHNHYTQKSLSLSPGKS GGGSGGGSGGGSGGAPTSSSTKKTQLQLEHLLLDLQ MILNGINNYKNPKLTRMLTFKFYMPKKATELKHLQ CLEEELKPLEEVLNLAQSKNFHLRPRDLISNINVIVL ELKGSETTFMCEYADETATIVEFLNRWITFSQSIISTL T |
| 60 | Humanized anti-PD-1, IgG4 heavy chain 1, knob Fc without IL-2 C125S | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSSASTKGPSVFPLAPCSRSTSESTAALGCLVKD YFPEPVTVSWNSGALTSGVHTFPAVLQSSGLYSLSSVVT VPSSSLGTKTYTCNVDHKPSNTKVDKRVESKYGPPCPP CPAPEFLGGPSVFLFPPKPKDTLMISRTPEVTCVVVDVS QEDPEVQFNWYVDGVEVHNAKTKPREEQFNSTYRVVS VLTVLHQDWLNGKEYKCKVSNKGLPSSIEKTISKAKGQ PREPRVYTLPPSQEEMTKNQVSLWCLVKGFYPSDIAVE WESNGQPENNYKTTPPVLDSDGSFFLYSRLTVDKSRWQ EGNVFSCSVMHEALHNHYTQKSLSLSPGK |
| 61 | Humanized anti-PD-1, IgG4 heavy chain with CD25 L42I | QVQLVQSGAEVKKPGASVKVSCKASGYTFTGYWMHW VRQAPGQGLEWIGMIHPNSDTTTYNEKFKNRVTMTRD TSISTAYMELSRLRSDDTAVYYCAGTDQAAWFAFWGQ GTTVTVSSASTKGPSVFPLAPCSRSTSESTAALGCLVKD YFPEPVTVSWNSGALTSGVHTFPAVLQSSGLYSLSSVVT VPSSSLGTKTYTCNVDHKPSNTKVDKRVESKYGPPCPP CPAPEFLGGPSVFLFPPKPKDTLMISRTPEVTCVVVDVS QEDPEVQFNWYVDGVEVHNAKTKPREEQFNSTYRVVS VLTVLHQDWLNGKEYKCKVSNKGLPSSIEKTISKAKGQ PREPQVYTLPPSQEEMTKNQVSLTCLVKGFYPSDIAVEW ESNGQPENNYKTTPPVLDSDGSFFLYSRLTVDKSRWQE GNVFSCSVMHEALHNHYTQKSLSLSPGKGGGGSGGGG SGGGGSGGGGSELCDDDPPEIPHATFKAMAYKEGTM LNCECKRGFRRIKSGSIYMLCTGNSSHSSWDNQCQC |
| | | TSSATRNTTKQVTPQPEEQKERKTTEMQSPMQPVD QASLPGHCREPPPWENEATERIYHFVVGQMVYYQC VQGYRALHRGPAESVCKMTHGKTRWTQPQLICTG |

## Claims

1. A fusion protein comprising at least one immunocyte antigen-binding domain, an Fc domain consisting of two Fc chains, and a cytokine function domain, wherein the cytokine function domain comprises α-subunit of interleukin-2 receptor (IL-2Rα) polypeptide or α-subunit of interleukin-15 receptor (IL-15Rα) polypeptide.

2. The fusion protein according to Claim 1, wherein the cytokine function domain comprises IL-2Rα polypeptide and IL-2 polypeptide, and said IL-2Rα polypeptide and IL-2 polypeptide are not covalently bonded to each other and are each individually linked to a different Fc chain of the Fc domain.

3. The fusion protein according to Claim 1, wherein the cytokine function domain is a complex comprising IL-2Rα polypeptide and IL-2 polypeptide, and said IL-2Rα polypeptide and IL-2 polypeptide in the complex are covalently bonded to each other and said complex is connected to one of the Fc chains of the Fc domain via IL-2Rα polypeptide or IL-2 polypeptide.

4. The fusion protein according to any one of Claims 1 to 3, wherein the IL-2Rα polypeptide is the IL-2Rα polypeptide having an amino acid sequence of SEQ ID NO: 3 or an IL-2-binding fragment, extracellular domain, or sushi domain thereof, and may comprise at least one amino acid modification that enhances the binding to the binding ligand, IL-2 polypeptide.

5. The fusion protein according to Claim 4, wherein the IL-2Rα polypeptide comprises at least one amino acid substitution selected from L2D, L2E, L2Q, M25L, M25I, N27A, N27T, N27I, N27Y, S39K, L42F, L42I, L42A, L42V, L45R, N57E, I118R, H120A, H120D, H120K, H120Y, H120L, H120W, H120R, and K153G based on the amino acid numbering of SEQ ID NO: 3.

6. The fusion protein according to Claim 4, wherein the IL-2Rα polypeptide comprises an amino acid substitution of L42I based on the amino acid numbering of SEQ ID NO: 3 or comprises the amino acid sequence of SEQ ID NO: 5.

7. The fusion protein according to Claim 2 or 3, wherein the IL-2 polypeptide comprises an amino acid substitution of C125S based on the amino acid numbering of SEQ ID NO: 1 or at least one amino acid modification that enhances the binding to the IL-2Rα polypeptide, or the IL-2 polypeptide is circularly permutated.

8. The fusion protein according to Claim 7, wherein the IL-2 polypeptide comprises an amino acid substitution of T37K based on the amino acid numbering of SEQ ID NO: 1.

9. The fusion protein according to Claim 1, wherein the cytokine function domain comprises IL-15Rα polypeptide and IL-15 polypeptide, and said IL-15Rαpolypeptide and IL-15 polypeptide are each individually linked to a different Fc chain of the Fc domain.

10. The fusion protein according to Claim 1, wherein the cytokine function domain is a complex comprising IL-15Rα polypeptide and IL-15 polypeptide, and said IL-15Rα polypeptide and IL-15 polypeptide in the complex are covalently bonded to each other and said complex is connected to one of the Fc chains of the Fc domain via IL-15Rα polypeptide or IL-15 polypeptide.

11. The fusion protein according to any one of Claims 1, 9, and 10, wherein the IL-15Rα polypeptide is the IL-15Rα polypeptide having an amino acid sequence of SEQ ID NO: 7 or an IL-15-binding fragment, extracellular domain, or sushi domain thereof, and may comprise at least one amino acid modification that enhances the binding to the binding ligand, IL-15 polypeptide.

12. The fusion protein according to Claim 9 or 10, wherein the IL-15 polypeptide comprises at least one amino acid modification that enhances the binding to the IL-15Rα polypeptide, or is circularly permutated.

13. The fusion protein according to Claim 1, wherein the immunocyte antigen-binding domain comprises a Fab molecule of an antibody that specifically binds to at least one T cell target antigen or an antigen-binding fragment thereof.

14. The fusion protein according to Claim 13, further comprising a Fab molecule of an antibody that specifically binds to at least one tumor cell target antigen or an antigen-binding fragment thereof.

15. The fusion protein according to Claim 13, wherein the immunocyte antigen-binding domain is a Fab molecule of an antibody that specifically binds to the PD-1 (programmed cell death-1) protein or an antigen-binding fragment thereof.

16. The fusion protein according to Claim 15, wherein the immunocyte antigen-binding domain is a Fab molecule of the pembrolizumab or nivolumab antibody or an antigen-binding fragment thereof, or a Fab molecule of an antibody or an antigen-binding fragment thereof that binds to the same epitope as or competes with the pembrolizumab or nivolumab antibody or an antigen-binding fragment thereof.

17. The fusion protein according to Claim 15, wherein the immunocyte antigen-binding domain is a Fab molecule of an anti-PD-1 antibody or an antigen-binding fragment thereof comprising
heavy chain complementarity-determining region 1 (HCDR1) comprising the amino acid sequence of SEQ ID NO: 9,
HCDR2 comprising an amino acid sequence selected from the group consisting of SEQ ID NOs: 10 to 15,
HCDR3 comprising an amino acid sequence selected from the group consisting of SEQ ID NOs: 16 to 31,
light chain complementarity-determining region 1 (LCDR1) comprising an amino acid sequence selected from the group consisting of SEQ ID NOs: 32 to 35,
LCDR2 comprising the amino acid sequence of SEQ ID NO: 36, and
LCDR3 comprising the amino acid sequences of SEQ ID NO: 37.

18. The fusion protein according to Claim 15, wherein the immunocyte antigen-binding domain comprises a Fab molecule of an anti-PD-1 antibody or an antigen-binding fragment thereof comprising complementarity determining region (CDR) 1, CDR2, and CDR3 of a heavy chain variable region comprising the amino acid sequence of SEQ ID NO: 38, 40, 42, or 44, and CDR1, CDR2, and CDR3 of a light chain variable region comprising the amino acid sequence of SEQ ID NO: 39, 41, 43, or 45.

19. The fusion protein according to Claim 17 or 18, wherein the immunocyte antigen-binding domain is a Fab molecule of an anti-PD-1 antibody or an antigen-binding fragment thereof comprising
a heavy chain variable region comprising an amino acid sequence having at least 75%, 80%, 85%, 90%, 95%, 96%, 97%, 98%, or 99% sequence identity to SEQ ID NO: 38, 40, 42, or 44 and
a light chain variable region comprising an amino acid sequence having at least 75%, 80%, 85%, 90%, 95%, 96%, 97%, 98%, or 99% sequence identity to SEQ ID NO: 39, 41, 43, or 45.

20. The fusion protein according to Claim 1, wherein the Fc domain is an IgG class Fc domain or an IgG1, IgG2, IgG3, or IgG4 subclass Fc domain; and when the Fc domain is an IgG4 Fc domain, it may comprise an amino acid substitution of S228P (based on the EU numbering system as in Kabat).

21. The fusion protein according to Claim 1, wherein the Fc domain comprises at least one amino acid modification that reduces the binding to Fc receptors or the effector function, or the Fc domain lacks CH2 domains.

22. The fusion protein according to Claim 21, wherein the Fc domain comprises an amino acid modification(s) at one or more positions selected from E233, L234, L235, G236, G237, N297, L328, P329, and P331 (based on EU numbering system as in Kabat).

23. The fusion protein according to Claim 1, wherein the Fc domain comprises a modification that promotes the formation of a heterodimer between the two Fc chains.

24. The fusion protein according to Claim 23, wherein the modification is a knob-into-hole modification comprising a knob modification in a chain of the Fc domain and a hole modification in the other Fc chain.

25. The fusion protein according to Claim 24, wherein the knob modification comprises an amino acid substitution of T366W, and the hole modification comprises amino acid substitutions of T366S, L368A, and Y407V, based on the EU numbering system of Kabat.

26. The fusion protein according to Claim 25, wherein the knob chain of the Fc domain further comprises an amino acid substitution of S354C, and the other chain further comprises an amino acid substitution of Y349C.

27. The fusion protein according to any one of Claims 24 to 26, wherein an Fc chain of the Fc domain further comprises an amino acid substitution of K360E, and the other Fc chain further comprises an amino acid substitution of Q347R,
or an Fc chain of the Fc domain further comprises amino acid substitutions of K360E and Q347E, and the other Fc chain further comprises an amino acid substitution of Q347R or amino acid substitutions of K360R and Q347R.

28. The fusion protein according to Claim 1, wherein the cytokine function domain is linked to the Fc domain via a linker.

29. The fusion protein according to Claim 28, wherein the linker is a peptide linker.

30. The fusion protein according to Claim 29, wherein the linker is a peptide of a general formula (GX)n, (GGGX)n, (XGGG)nXGG, or (GGGGX)n, in which X is A or S, and n is a natural number from 1 to 4.

31. The fusion protein according to any one of Claims 13 to 19, wherein the antigen-binding fragment is scFv, (scFv)₂, Bis-scFv, dsFv, (dsFv)₂, Fv, dsFv-dsFv', diabody, ds-diabody, triabody, tetrabody, nanobody, domain antibody, single domain antibody (sdAb), or bivalent domain antibody.

32. A polynucleotide encoding the fusion protein according to any one of Claims 1 to 3, 9, and 10.

33. An expression vector comprising the polynucleotide according to Claim 32.

34. A host cell comprising the polynucleotide of Claim 32 or an expression vector comprising the same.

35. A method of producing the fusion protein according to any one of Claims 1 to 3, 9, and 10 comprising a step of culturing a host cell containing an expression vector that comprises a polynucleotide encoding the fusion protein according to any one of Claims 1 to 3, 9, and 10.

36. The method according to Claim 35, wherein the expression vector comprises
a first expression vector comprising a polynucleotide sequence encoding the amino acid sequence of SEQ ID NO: 51 and a polynucleotide sequence encoding the amino acid sequence of SEQ ID NO: 57 and
a second expression vector comprising a polynucleotide sequence encoding the amino acid sequence of SEQ ID NO: 51 and a polynucleotide sequence encoding the amino acid sequence of SEQ ID NO: 58.

37. The method according to Claim 36, wherein the first and second expression vectors are co-expressed in a host cell to obtain the fusion protein.

38. A pharmaceutical composition comprising the fusion protein according to any one of Claims 1 to 3, 9, and 10 for the prevention, amelioration, or treatment of tumor, cancer, metastatic tumor, metastatic cancer, or infectious disease.
